# EUROPEAN PATENT APPLICATION

(11) **EP 1 344 832 A1**
(43) Date of publication of application: **17.09.2003**
(21) Application number: 02005996.0
(22) Date of filing: 15.03.2002
(51) Int. Cl.: C12Q 1/68

(54) **Methods and nucleic acids for the analysis of methylation within the gene melastatin**

(71) Applicant: Epigenomics AG, 10435 Berlin (DE)
(72) Inventor: Olek, Alexander, 10407 Berlin (DE)
(74) Representative: Schohe, Stefan

(57) **Abstract**

The present invention provides methods for the analysis of the methylation status of the gene Melastatin. Furthermore the invention discloses genomic and chemically modified nucleic acid sequence derived from the gene Melastatin, as well as oligonucleotides and/or PNA-oligomers for analysis of cytosine methylation patterns within said gene. The present invention is based on the discovery that genetic and epigenetic parameters, in particular, the cytosine methylation patterns, of the gene Melastatin are particularly suitable for the diagnosis and/or therapy of disease conditions, in particular dermal cell proliferative disorders.

## Description

### Background

Skin cancer is the most common of all cancers. About 44,000 people were diagnosed with melanoma in the year 2000 in the United States alone. The American Cancer Society estimates that over 51,000 patients will be diagnosed with melanoma in the year 2001 of which 7,800 cases will be fatal. The incidence of melanoma is increasing at the rate of 2.5 to 4% per year and metastatic melanoma is lethal in 86% of the cases.

Melanomas are malignant changes to melanin producing cells. They account for about 4% of skin cancer cases, but account for some 79% of deaths from skin cancer. The occurrence of severe sunburn during childhood leading to melanoma in adults is relatively frequent. (Herlyn M, Satyamoorthy K. Molecular biology of cutaneous melanoma. In Cancer: principles and practice of oncology. DeVita VT, Hellman, S, Rosenberg, SA, eds. 6th edition. Philadelphia: Lippincott Williams and Wilkins, 2001:2003-2012.; Noonan FP, Recio JA, Takayama H, Dury P, Anver MR, Rush WL et al. (2001) Neonatal sunburn and melanoma in mice. Nature 413:271-272)

The role of genetics in the development of melanomas has been observed, however the cellular mechanisms associating the development of melanomas have not been identified. Several genes that do cause inherited melanoma have been identified. These include CDKN2A (also known as p16 and INK4A) and CDK4. Less than 5 percent of melanoma cases, however, are due to mutations in these genes. A11 of these genetic conditions clearly interact with the environment, and in particular with sunlight exposure, to cause melanoma.

The treatment options for metastatic melanoma are not optimal as the tumors are radioresistant, chemoresistant and hence resistant to apoptosis. The pathways and molecules that are responsible and utilized for conventional therapeutic manipulations do not appear to be involved. For example, oncogenes such as myc and ras have been implicated in radioresistance but activation of these genes does not correlate with the response to therapy. The search for overexpression of drug resistant genes has also had limited success (Serrone L, Hersey P. (1999) The chemoresistance of human malignant melanoma: an update. Melanoma Res 9: 51-58).

The high number of deaths from melanoma are largely resulting from the cancerous cells metastasising, or spreading. A distinguishing feature of malignant cells is their capacity to invade surrounding normal tissues and metastasize through the blood and lymphatic systems to distant organs. It has long been recognized that cancer metastasis is a highly complicated phenomenon requiring the interaction of many different types of cells, connective tissue, and blood vessel components within different organs.

People who develop melanomas have quite varying rates of survival, and determining who is most at risk is a somewhat subjective assessment, generally dependent on measuring the thickness of the tumour.

Duncan *et al.* (J Clin Oncol 15:568-576, 2001) investigated the expression of a gene called Melastatin in both cancerous and non-cancerous cells. Melastatin is a gene that is specifically expressed in melanocytes and has been implicated in the severity of melanoma. Investigation into the gene Melastatin (also known as MLSN1), suggest that the molecular steps that precede its actual production may be used to predict whether or not the type of skin lesions involved in melanoma will be metastatic or not. When the level of messenger RNA (mRNA) for MLSN1 is reduced, the chances increase sharply that the melanoma will be metastatic. When there are normal amounts of MLSN mRNA, the melanoma is much more likely to be benign and/or the lesions less severe (Deeds J et al: Hum Pathol 31:1346-1356, 2000; Duncan LM et al: J Clin Oncol 15:568-576, 2001). The loss of gene expression increases the risk of cancer metastasis over time more than six-fold.

A previous study published in November 2000 (Deeds J et al: Hum Pathol 31:1346-1356, 2000) also noted that Melastatin gene expression correlated with melanoma tumour progression, thickness, and the potential for metastasis in normal skin and benign melanocytic nevi. (Moles.) Hence detecting the level of melastatin in patient tissue samples can help determine whether a patient has, or is at risk of, developing metastatic melanoma, a serious form of skin cancer.

Therefore, expression of a single gene may provide to be a significant prognostic indicator as to melanoma development. This is of particular importance as 95% of melanoma cases are curable if diagnosed early and surgically excised.

US 6,312,909 (Millennium Pharmaceuticals Inc.) covers the use of a gene 'fohy030', a homolog of the melastatin gene, for detection of mRNA levels in tissue samples in order to diagnose, monitor or treat tumor progression. The patent covers methods based on the detection or quantitation of fohy030 mRNA or the correlating polypeptides. Melastatin belongs to a subfamily of the TRP (transient receptor potential) superfamily. The subfamily consists of channel-like proteins. They mediate Ca²⁺ ions entry when expressed in presenelin 1 expressing human embryonic kidney cells (HEK293). However, melastatin is alternatively spliced, and one of the major isoforms is predicted to encode a short protein that includes only the N-terminus but not any transmembrane domain (Fang, D and Setaluri, V (2000) Biochem. Biophys. Res. Commun. 279: 53-61). Consequently it is incapable of functioning independently as an ion channel, but could be shown to be involved in the regulation of melastatin. It has been proposed that the interaction of the short form and the full length form of melastatin (MLSN) somehow control the translocation of the full length melastatin and through this provides a mode for regulating ion channel activity (Xu et al. (2001) Regulation of melastatin, a TRP-related protein, through interaction with a cytoplasmic isoform. PNAS 98: 10692-10697).

For a number of genes involved in cancer it has been shown that methylation of the correlating promoter region is responsible for the regulation of gene expression. For example, in prostate carcinoma patients the promoter of the gene GSTP1 (glutathionyltransferase P1) is hypermethylated and that way causes GSTP1 expression to be silenced. The methylated DNA can be found in blood, urine or ejaculate samples from prostate carcinoma patients. In a recent study 94% of the tumor DNA samples were methylated, 72% of the plasma or serum samples, 50% of ejaculate samples and 36% of urine samples (after prostatic massage in order to release prostatic secretions) from patients with prostate cancer whereas no methylation was detected in samples from the control group (Cairns et al. (2001) Clin Cancer Res 7: 2727-2730).

5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing since 5-methylcytosine has the same base pairing behaviour as cytosine. Moreover, the epigenetic information carried by 5-methylcytosine is completely lost during PCR amplification.

A relatively new and currently the most frequently used method for analyzing DNA for 5-methylcytosine is based upon the specific reaction of bisulfite with cytosine which, upon subsequent alkaline hydrolysis, is converted to uracil which corresponds to thymidine in its base pairing behavior. However, 5-methylcytosine remains unmodified under these conditions. Consequently, the original DNA is converted in such a manner that methylcytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited. In terms of sensitivity, the prior art is defined by a method which encloses the DNA to be analyzed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyze individual cells, which illustrates the potential of the method. However, currently only individual regions of a length of up to approximately 3000 base pairs are analyzed, a global analysis of cells for thousands of possible methylation events is not possible. However, this method cannot reliably analyze very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res. 1998, 26, 2255.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridization has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun; 16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705 and WO 95/15373.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionization of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionization Mass Spectrometry. Current Innovations and Future Trends. 1995, 1; 147-57). The sensitivity to nucleic acids is approximately 100 times worse than to peptides and decreases disproportionally with increasing fragment size. For nucleic acids having a multiply negatively charged backbone, the ionization process via the matrix is considerably less efficient. In MALDI-TOF spectrometry, the selection of the matrix plays an eminently important role. For the desorption of peptides, several very efficient matrixes have been found which produce a very fine crystallisation. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8): 1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA, as the raw material source for achieving the bisulfite sequence, is obtained from DNA of cell, tissue or other test samples from (potential) patients using standard methods. This standard methodology is found in references given within or in Olek et al., WO 99/28498 and in Sambrook, Fritsch and Maniatis eds., Molecular Cloning: A Laboratory Manual, 1989.

### Description

The present invention provides methods for the analysis of the methylation status of the gene Melastatin. Furthermore the invention discloses genomic and chemically modified nucleic acid sequence derived from the gene Melastatin, as well as oligonucleotides and/or PNA-oligomers for analysis of cytosine methylation patterns within said gene. The present invention is based on the discovery that genetic and epigenetic parameters, in particular, the cytosine methylation patterns, of the gene Melastatin are particularly suitable for the diagnosis and/or therapy of disease conditions, in particular dermal cell proliferative disorders.

This objective is achieved according to the present invention using a chemically modified nucleic acid containing a sequence of at least 18 bases in length according to one of Seq. ID No. 2 to Seq. ID No. 4 and sequences complementary thereto, or a sequence of at least 18 bases in length according to Seq. ID. No. 1 and sequences complementary thereto. Seq. ID No.1 discloses a fragment of the promoter region of the gene Melastatin, wherein said fragment contains CpG rich regions. Said fragment of the gene Melastatin was heretofore not associated with methylation analysis. Due to the degeneracy of the genetic code, the sequence as identified in Seq. ID No. 1 should be interpreted so as to include all substantially similar and equivalent sequences within the promoter region of a gene which encodes a polypeptide with the biological activity of Melastatin. Furthermore Seq. ID No. 2 through 5 provide chemically modified version of the nucleic acid according to Seq. ID No. 1 wherein the chemical modification of said sequence results in the synthesis of a sequence that is unique and distinct from Seq. ID No. 1. The nucleic acid molecules according to Seq. ID No. 1 to Seq. ID No. 5 could heretofore not be connected with the ascertainment of genetic and epigenetic parameters.

The object of the present invention is further achieved by an oligonucleotide or oligomer for detecting the cytosine methylation state within pretreated DNA or genomic DNA according to Seq. ID No. 1 to Seq. ID No. 5. Said oligonucleotide or oligomer containing at least one base sequence having a length of at least 9 nucleotides which hybridizes to a pretreated nucleic acid sequence according to Seq. ID No. 2 to Seq. ID No. 5 and sequences complementary thereto or to a genomic sequence comprising Seq. ID No. 1 and sequences complementary thereto. The oligomer probes according to the present invention constitute important and effective tools which, for the first time, make it possible to ascertain the genetic and epigenetic parameters of the gene Melastatin. The base sequence of the oligomers preferably contain at least one CpG dinucleotide. The probes may also exist in the form of a PNA (peptide nucleic acid) which has particularly preferred pairing properties. Particularly preferred are oligonucleotides according to the present invention in which the cytosine of the CpG dinucleotide is within the middle third of the oligonucleotide, i.e. wherein the oligonucleotide is 13 bases in length the CpG dinucleotide is positioned within the 5^{th}- 9^{th} nucleotide from the 5'-end.

The oligomers according to the present invention are normally used in so called "sets" which contain at least one oligomer for analysis of each of the CpG dinucleotides of the sequences of a pretreated nucleic acid according to Seq. ID No. 2 to Seq. ID No. 5 and sequences complementary thereto and/or to a genomic sequence comprising Seq. ID No. 1 and sequences complementary thereto. Preferred is a set which contains at least one oligomer for each of the CpG dinucleotides within the gene Melastatin in both the pretreated and genomic versions of said gene, Seq. ID No. 2 through 5 and Seq. ID No. 1, respectively.

Moreover, the present invention makes available a set of at least two oligonucleotides which can be used as so-called "primer oligonucleotides" for amplifying DNA sequences of one of Seq. ID No. 1 to Seq. ID No. 5 and sequences complementary thereto, or segments thereof.

In the case of the sets of oligonucleotides according to the present invention, it is preferred that at least one and more preferably all members of the set of oligonucleotides is bound to a solid phase.

The present invention moreover relates to a set of at least 3 n (oligonucleotides and/or PNA-oligomers) used for detecting the cytosine methylation state in pretreated genomic DNA (Seq. ID No. 2 to Seq. ID No. 5 and sequences complementary thereto) and genomic DNA (Seq. ID No. 1 and sequences complementary thereto). These probes enable diagnosis and/or therapy of genetic and epigenetic parameters of diseases associated with the gene Melastatin, including dermal cell proliferative disorders. The set of oligomers may also be used for detecting single nucleotide polymorphisms (SNPs) in pretreated genomic DNA (Seq. ID No. 2 to Seq. ID No. 5, and sequences complementary thereto) and genomic DNA (Seq. ID No. 1, and sequences complementary thereto) .

According to the present invention, it is preferred that an arrangement of different oligonucleotides and/or PNA-oligomers (a so-called "array") made available by the present invention is present in a manner that it is likewise bound to a solid phase. This array of different oligonucleotide- and/or PNA-oligomer sequences can be characterised in that it is arranged on the solid phase in the form of a rectangular or hexagonal lattice. The solid phase surface is preferably composed of silicon, glass, polystyrene, aluminium, steel, iron, copper, nickel, silver, or gold. However, nitrocellulose as well as plastics such as nylon which can exist in the form of pellets or also as resin matrices may also be used.

Therefore, a further subject matter of the present invention is a method for manufacturing an array fixed to a carrier material for analysis in connection with diseases associated with the gene Melastatin in which method at least one oligomer according to the present invention is coupled to a solid phase. Methods for manufacturing such arrays are known, for example, from US Patent 5,744,305 by means of solid-phase chemistry and photolabile protecting groups.

A further subject matter of the present invention relates to a DNA chip for the analysis of diseases associated with the gene Melastatin. DNA chips are known, for example, for US Patent 5,837,832.

Moreover, a subject matter of the present invention is a kit which may be composed, for example, of a bisulfite-containing reagent, a set of primer oligonucleotides containing at least two oligonucleotides whose sequences in each case corresponds or are complementary to an 18 base long segment of the base sequences specified in the appendix (Seq. ID No. 1 to Seq. ID No. 5 and sequences complementary thereto), oligonucleotides and/or PNA-oligomers as well as instructions for carrying out and evaluating the described method. However, a kit along the lines of the present invention can also contain only part of the aforementioned components.

The present invention also makes available a method for ascertaining genetic and/or epigenetic parameters of the gene Melastatin within a subject by analysing cytosine methylations and single nucleotide polymorphisms. Said method comprising contacting a nucleic acid comprising one or more sequences from the group comprising Seq. ID No.1 through Seq. ID No. 5 in a biological sample obtained from said subject with at least one reagent or a series of reagents, wherein said reagent or series of reagents, distinguishes between methylated and non methylated CpG dinucleotides within the target nucleic acid.

In a preferred embodiment said method including the following steps:

In the first step of the method, a genomic DNA sample is chemically treated in such a manner that cytosine bases which are unmethylated at the 5'-position are converted to uracil, thymine, or another base which is dissimilar to cytosine in terms of hybridization behaviour. This will be understood as ' 'pretreatment' hereinafter.

The genomic DNA to be analysed is preferably obtained from usual sources of DNA such as cells or cell components, for example, cell lines, biopsies, blood, sputum, tissue embedded in paraffin such as tissue from histologic object slides, or combinations thereof.

The above described treatment of genomic DNA is preferably carried out with bisulfite (hydrogen sulphite, disulfite) and subsequent alkaline hydrolysis which results in a conversion of non-methylated cytosine nucleobases to uracil or to another base which is dissimilar to cytosine in terms of base pairing behaviour.

Fragments of the pretreated DNA are amplified, using sets of primer oligonucleotides according to the present invention, and a, preferably heat-stable polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

In a preferred embodiment of the method, the set of primer oligonucleotides includes at least two oligonucleotides whose sequences are each reverse complementary or identical to an at least 18 base-pair long segment of the base sequences specified in the appendix (Seq. ID No. 1 to Seq. ID No. 5 and sequences complementary thereto).

According to the present invention, it is preferred that at least one, more preferably all, primer oligonucleotides used are bonded to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminium, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualised by means of matrix assisted laser desorption/ionization mass spectrometry (MALDI) or using electron spray mass spectrometry (ESI).

The amplificates obtained in the second step of the method are subsequently hybridized to an array or a set of oligonucleotides and/or PNA probes. In this context, the hybridization takes place in the manner described in the following. The set of probes used during the hybridization is preferably composed of at least 3 oligonucleotides or PNA-oligomers. In the process, the amplificates serve as probes which hybridise to oligonucleotides previously bonded to a solid phase. The non-hybridized fragments are subsequently removed. Said oligonucleotides contain at least one base sequence having a length of at least 9 nucleotides which is reverse complementary or identical to a segment of the base sequences specified in the appendix, the segment containing at least one CpG dinucleotide. The cytosine of the CpG dinucleotide is the 5^{th} to 9^{th} nucleotide from the 5'-end of the 13-mer. One oligonucleotide exists for each CpG dinucleotide. Said PNA-oligomers contain at least one base sequence having a length of 9 nucleotides which is reverse complementary or identical to a segment of the base sequences specified in the appendix, the segment containing at least one CpG dinucleotide. The cytosine of the CpG dinucleotide is the 4^{th} to 6^{th} nucleotide seen from the 5'-end of the 9-mer . In a preferred embodiment one oligonucleotide exists for each CpG dinucleotide.

In the fourth step of the method, the non-hybridized amplificates are removed.

In the final step of the method, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualised by means of matrix assisted laser desorption/ionization mass spectrometry (MALDI) or using electron spray mass spectrometry (ESI).

The produced fragments may have a single positive or negative net charge for better detectability in the mass spectrometer.

A further embodiment of the method according to the invention is a method for the analysis of the methylation status of genomic DNA without the need for pretreatment.

In the first step of the method the genomic DNA sample must be isolated from tissue or cellular sources. Such sources may include cell lines, histological slides, body fluids, or tissue embedded in paraffin. Extraction may be by means that are standard to one skilled in the art, these include the use of detergent lysates, sonification and vortexing with glass beads. Once the nucleic acids have been extracted the genomic double stranded DNA is used in the analysis.

In a preferred embodiment the DNA may be cleaved prior to the treatment, this may be any means standard in the state of the art, in particular with restriction endonucleases. In the second step, the DNA is then digested with one or more methylation sensitive restriction enzymes. The digestion is carried out such that hydrolysis of the DNA at the restriction site is informative of the methylation status of a specific CpG dinucleotide.

In the third step which is optional but a preferred embodiment the restriction fragments are amplified. This is preferably carried out using a polymerase chain reaction.

In the final step the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Suitable labels for use on the detection of the digested nucleic acid fragments include fluorophore labels, radionuclides and mass labels as described above.

The oligomers according to the present invention or arrays thereof as well as a kit according to the present invention are intended to be used for the diagnosis and/or therapy of diseases associated with the gene Melastatin. According to the present invention, the method is preferably used for the diagnosis and/or therapy of important genetic and/or epigenetic parameters within said aforementioned gene.

The methods according to the present invention are used, for example, for the diagnosis and/or therapy of dermal cell proliferative disorders.

The nucleic acids according to the present invention Seq. ID No. 1 to Seq. ID No. 5 and sequences complementary thereto can be used for the diagnosis and/or therapy of genetic and/or epigenetic parameters of the gene Melastatin.

The present invention moreover relates to a method for manufacturing a diagnostic agent and/or therapeutic agent for the diagnosis and/or therapy of diseases associated with the gene Melastatin by analysing methylation patterns of said gene, the diagnostic agent and/or therapeutic agent being characterised in that at least one nucleic acid according to the present invention is used for manufacturing it, possibly together with suitable additives and auxiliary agents.

A further subject matter of the present invention relates to a diagnostic agent and/or therapeutic agent for diseases associated with the gene Melastatin by analysing methylation patterns of said gene, the diagnostic agent and/or therapeutic agent containing at least one nucleic acid according to the present invention, possibly together with suitable additives and auxiliary agents.

The present invention moreover relates to the diagnosis and/or prognosis of events which are disadvantageous to patients or individuals in which important genetic and/or epigenetic parameters within the gene Melastatin may be used as markers. Said parameters obtained by means of the present invention may be compared to another set of genetic and/or epigenetic parameters, the differences serving as the basis for a diagnosis and/or prognosis of events which are disadvantageous to patients or individuals.

In the context of the present invention the term "hybridization" is to be understood as a bond of an oligonucleotide to a completely complementary sequence along the lines of the Watson-Crick base pairings in the sample DNA, forming a duplex structure. To be understood by "stringent hybridization conditions" are those conditions in which a hybridization is carried out at 60°C in 2.5 x SSC buffer, followed by several washing steps at 37°C in a low buffer concentration, and remains stable.

In the context of the present invention, "genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

In the context of the present invention, "epigenetic parameters" are, in particular, cytosine methylations and further chemical modifications of DNA bases of genes associated with DNA adducts and sequences further required for their regulation. Further epigenetic parameters include, for example, the acetylation of histones which, however, cannot be directly analysed using the described method but which, in turn, correlates with the DNA methylation.

The present invention shall now be further described in the following example. In the attached Sequence Listing,

Seq. ID No.1 discloses a fragment of the promoter region of the gene Melastatin, wherein said fragment contains CpG rich regions.

Seq. ID No. 2 through 5 provide chemically modified version of the nucleic acid according to Seq. ID No. 1 wherein the chemical modification of said sequence results in the synthesis of a sequence that is unique and distinct from Seq. ID No. 1.

Seq. ID No. 6 through 9 provide specific primer sequences as used in Example 1.

### Example 1: Methylation analysis of the MLSN1 gene.

The following example relates to a fragment of the gene MLSN1 in which a specific CG-position is to be analyzed for methylation.

In the first step, a genomic sequence is treated using bisulfite (hydrogen sulfite, disulfite) in such a manner that all cytosines which are not methylated at the 5-position of the base are modified in such a manner that a different base is substituted, said base exhibiting different base pairing behaviour to cytosine, while the cytosines methylated at the 5-position remain unchanged.

If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA is then used for the detection of methylated cytosines. In the second method step, the treated DNA sample is diluted with water or an aqueous solution. Preferably, the DNA is subsequently desulfonated. In the third step of the method, the DNA sample is amplified in a polymerase chain reaction, preferably using a heat-resistant DNA polymerase. In the present case, cytosines of the gene MLSN1 are analyzed. To this end, a defined fragment having a length of 451 bp is amplified with the specific primer oligonucleotides TAGAGTTTTGAAGTTGGAGGTT (Seq. ID No. 6) and TCACTTCCCCATATAATCTTTC (Seq. ID No. 7).

The amplificate serves as a sample which hybridizes to an oligonucleotide previously bound to a solid phase, forming a duplex structure, for example GGGTAATTCGGGGTATTT (Seq. ID No. 8, the cytosine to be detected being located at position 397 of the amplificate. The detection of the hybridization product is based on Cy3 and Cy5 fluorescently labelled primer oligonucleotides which have been used for the amplification. A hybridization reaction of the amplified DNA with the oligonucleotide takes place only if a methylated cytosine was present at this location in the bisulfite-treated DNA. Thus, the methylation status of the specific cytosine to be analyzed is inferred from the hybridization product.

In order to verify the methylation status of the position, a sample of the amplificate is further hybridized to another oligonucleotide previously bonded to a solid phase. Said olignonucleotide is identical to the oligonucleotide previously used to analyze the methylation status of the sample, with the exception of the position in question. At the position to be analysed said oligonucleotide comprises a thymine base as opposed to a cytosine base i.e GGGTAATTTGGGGTATTT (Seq. ID No. 9). Therefore, the hybridisation reaction only takes place if an unmethylated cytosine was present at the position to be analysed.

## Claims

1. A method for detecting the methylation state of cytosine bases within the gene Melastatin, said method comprising contacting a nucleic acid comprising one or more sequences from the group comprising Seq. ID No.1 through Seq. ID No. 5 with at least one reagent or a series of reagents, wherein said reagent or series of reagents, distinguishes between methylated and non methylated CpG dinucleotides within the nucleic acid.

2. A nucleic acid molecule comprising a sequence at least 18 bases in length which hybridizes to or is identical to one of the sequences taken from the group comprising Seq. ID No. 1 to Seq. ID No. 5 and sequences complementary thereto.

3. An oligomer, in particular an oligonucleotide or peptide nucleic acid (PNA)-oligomer, said oligomer comprising in each case at least one base sequence having a length of at least 9 nucleotides which hybridizes to or is identical to one of the nucleic acid sequences according to Seq. ID No. 1 through Seq. ID No. 5.

4. The oligomer as recited in Claim 3; wherein the base sequence includes at least one CpG dinucleotide.

5. The oligomer as recited in Claim 4; **characterised in that** the cytosine of the CpG dinucleotide is located approximately in the middle third of the oligomer.

6. A set of oligomers, comprising at least two oligomers according to any of claims 3 to 5.

7. A set of oligomers as recited in Claim 6, comprising oligomers for detecting the methylation state of all CpG dinucleotides within one of the sequences according to Seq. ID No. 1 to Seq. ID No. 5 and sequences complementary thereto.

8. A set of at least two oligonucleotides as recited in one of Claims 3 through 7, which can be used as primer oligonucleotides for the amplification of DNA sequences of one of Seq. ID No. 1 to Seq. ID No. 5 and sequences complementary thereto.

9. A set of oligonucleotides as recited in one of Claims 6 to 8, **characterised in that** at least one oligonucleotide is bound to a solid phase.

10. Use of a set of oligonucleotides comprising at least three of the oligomers according to any of claims 3 through 9 for detecting the cytosine methylation state and/or single nucleotide polymorphisms (SNPs) within the sequences taken from the group comprising Seq. ID No. 1 to Seq. ID No. 5 and sequences complementary thereto.

11. A method for manufacturing an arrangement of different oligomers (array) fixed to a carrier material for analysing diseases associated with the methylation state of the CpG dinucleotides of the gene Melastatin wherein at least one oligomer according to any of the claims 3 through 9 is coupled to a solid phase.

12. An arrangement of different oligomers (array) obtainable according to claim 11.

13. An array of different oligonucleotide- and/or PNA-oligomer sequences as recited in Claim 12, **characterised in that** these are arranged on a solid phase in the form of a rectangular or hexagonal lattice.

14. The array as recited in any of the Claims 12 or 13, **characterised in that** the solid phase surface is composed of silicon, glass, polystyrene, aluminium, steel, iron, copper, nickel, silver, or gold.

15. A DNA- and/or PNA-array for analysing diseases associated with the methylation state of the gene Melastatin comprising at least one nucleic acid according to one of the preceding claims.

16. A method for determining the methylation state within at least one nucleic acid molecule according to Claim 1, **characterised in that** the following steps are carried out:
a) obtaining a biological sample containing genomic DNA
b) extracting the genomic DNA
c) converting cytosine bases which are unmethylated at the 5-position within said DNA sample, by chemical treatment, to uracil or another base which is dissimilar to cytosine in terms of hybridization behaviour;
d) amplifying fragments of the chemically pretreated genomic DNA using sets of primer oligonucleotides according to one of Claims 8 or 9;
e)identifying the methylation status of one or more cytosine positions.

17. The method as recited in Claim 16, **characterised in that** the chemical treatment is carried out by means of a solution of a bisulfite, hydrogen sulfite or disulfite.

18. A method for the analysis of methylation within at least one nucleic acid molecule according to Claim 1 comprising the following steps;
a) obtaining a biological sample containing genomic DNA
b) extracting the genomic DNA
c) digesting the genomic DNA comprising Seq. ID No. 1 with one or more methylation sensitive restriction enzymes
d) detection of the DNA fragments generated in the digest of step c)

19. A method according to Claim 18, wherein the DNA digest is amplified prior to Step d.

20. The method as recited in one of the Claims 16, 17 and 19, **characterised in that** more than ten different fragments having a length of 100 - 2000 base pairs are amplified.

21. The method as recited in one of the Claim 20, **characterised in that** the amplification of several DNA segments is carried out in one reaction vessel.

22. The method as recited in Claim 21, **characterised in that** the polymerase is a heat-resistant DNA polymerase.

23. The method as recited in Claim 22, **characterised in that** the amplification is carried out by means of the polymerase chain reaction (PCR).

24. The method as recited in one of the Claims 16, 17 and 19 through 23 **characterised in that** the nucleic acid amplificates carry a detectable label

25. The method as recited in Claim 24, **characterised in that** the labels of the amplificates are fluorescence labels.

26. The method as recited in Claim 24, **characterised in that** the labels of the amplificates are radionuclides.

27. The method as recited in Claim 24, **characterised in that** the labels of the amplificates are detachable molecule fragments having a typical mass which are detected in a mass spectrometer.

28. The method as recited in one of the Claims 16, 17 and 19 through 24, **characterised in that** the amplificates or fragments of the amplificates are detected in the mass spectrometer.

29. The method as recited in one of Claims 24, 27 or 28, **characterised in that** the produced fragments have a single positive or negative net charge for better detectability in the mass spectrometer.

30. The method as recited in one of the Claims 27, 28 and 29, **characterised in that** detection is carried out and visualized by means of matrix assisted laser desorption/ionization mass spectrometry (MALDI) or using electron spray mass spectrometry (ESI).

31. The method as recited in one of the Claims 16 through 30, **characterised in that** the genomic DNA is obtained from cells or cellular components which contain DNA, sources of DNA comprising, for example, cell lines, histological slides, biopsies, tissue embedded in paraffin and all possible combinations thereof.

32. A kit comprising a bisulfite (= disulfite, hydrogen sulfite) reagent as well as oligonucleotides and/or PNA-oligomers according to one of the Claims 3 through 10.

33. The use of a method according to Claim 1, a nucleic acid according to Claim 2, of an oligonucleotide or PNA-oligomer according to one of the Claims 3 through 5, of a kit according to Claim 32, of an array according to one of the Claims 11 through 15, of a set of oligonucleotides according to one of claims 6 through 10 or a method according to one of claims 16 through 31 for the characterisation, classification, differentiation, grading, staging, and/or diagnosis of dermal cell proliferative disorders, or the predisposition to dermal cell proliferative disorders.

34. The use of a method according to Claim 1, a nucleic acid according to Claim 2, of an oligonucleotide or PNA-oligomer according to one of the Claims 3 through 5, of a kit according to Claim 32, of an array according to one of the Claims 11 through 15, of a set of oligonucleotides according to one of claims 6 through 10 or a method according to one of claims 16 through 31 for the therapy of dermal cell proliferative disorders.


Sequence listing

<110> Epigenomics AG

<120> Melastatin

<160> 9

<210> 1
<211> 11097
<212> DNA
<213> Homo Sapiens

<400> 1
   agtagctggg attacaggca cccaccacca tggccggtta ttttttatat ttttagtaga 60
   gatgaggttt caccatgttg gccaggctgg tctcgaactc ctgacctcag gtgatccacc 120
   tgcctcagcc tcccaaagtg ctggaattgc aggcgtgagc tgggccaatt taaaattttc 180
   tagtagtcag ttttgaaaag taaatagaag caggtgaaaa aaattaataa cattctattc 240
   aacccaacat gttctaaata tttcaatgtg caatcgatta ttatcacaag tcttggcaat 300
   cccgtgtata ttttacattc ggagcatatc tcaattctga ctggctctgt ttccagcact 360
   ccatagccac gcgtggcctg tggccactgt gtgagacatc tcagctccca gttgcaggcc 420
   acactgcatg gaccctgggg aggaggggtg ttctcttggg gaaggatggg gctcccattg 480
   tttccatctt atctgaacct gaaaaagagc ttatttgtct agtgaagggc ccactgcctg 540
   gagagccact gactggcagc cggtcgggct ggccattggt gctgaccaca gagcctccac 600
   acctcccagc catggcagtg ccggccatca gctcacttcc ccatgtggtc tttccccact 660
   gctcccagac tgatttcaga tgccccggat tgccctgact gtgccttcag aattccggcc 720
   acgtagcacc ctccctcagc ccccggaagg gttcccgctg aagccgtgac tctctcaggc 780
   ctcaggagcc aacaccagtt caagaaagaa gactcgggag ccaggccagg gaggctgctg 840
   cggtcattgt gcgcccaggg gctgtgcgcg ctccagcaaa gcacgtggga gccactcacc 900
   agaccctttg cacgcccact ctgcggtgac agaccctgag agaaacgaat gaaagcaaag 960
   acagacccag aaaatggcct ggcacatgtg cttccgtcag aagttcagag attccctgag 1020
   ccccaggctg tctgccaggc attgggtggg ggagtgagcc cagcctccag cttcaggact 1080
   ctacatatgg gcagaacttg acaacctggg cactcccact ccaccccacc tgctctctgg 1140
   gcaagccacc ctctcacatg actcagagtc ctcatctgta aaatgggaac acccctaatt 1200
   gcccttacgt ccaatctgtc tgctcctctg tgagaagcgg gaggcggaca atgcccaaga 1260
   gcctcagatg ctggggaaga cccagcccct ctgcagagag ggatagttca gggtttgggt 1320
   tttttctttc ctcctgggct gagaaagctc atggaaagct ggaataacca cgcatactgt 1380
   tacagcaaat ccaacagagc tcccaccgtg gtggttttca ggtgactgtg gatgccaagc 1440
   aggcaagctc ctggtgaagg ggggagagca gggattagaa gcactcagaa ggggctgaga 1500
   gtcatgtggg gctcacactg catttgcagc tgggttcacc ctgacctcag gcccaactta 1560
   gatgaggaag gattagcagt aattagtgcc atgtgccgcc ttctcccagc tccccggggc 1620
   acgaacactg cccagctgat gaggggattc tgaaagaacc attatgtcca attgtctcaa 1680
   ttatgcaaac cctgctgaca tttccagcca gggaagggcg gctgggtggg agggggccat 1740
   ggcggggcca cttcaaagga aaagctctag ctcccctacc tctctcacat cctaaggctg 1800
   cctttgtggg attccacaca gaacagcctg gaagcttggg gccctggctt ccttttctgg 1860
   cctgggagtc aggtcatggg gccatcgctt cacagcaatc atgagggccc aggcccaagt 1920
   gctcacatgc tcctcatggg gactgctcct cttaaagggt gggccctcct cacccagctc 1980
   cctgccctgg ccaaggagga ggctgaaaga gcctgagctg tgccctctcc attccactgc 2040
   tgtggcaggt aagctcagat gcgggtaagc acaggtgaag ggaggtcagg tgcaggtatc 2100
   ctcaggtgtg ggtaaactca agcgtggaca aactcaggtg cagataacca cagatgcagt 2160
   taagctcagg tgttggtaac ctcaggtgca ggtaggctca ggtgcaagta agagcaggtg 2220
   caggcaagct caggtgtgct ttctggggcc tgttactcat ctcctcttga ggctggctgg 2280
   acaggaccct atggagcagc tggtgaacct gtgttcagcc tgggttccaa gaggctgtct 2340
   cagcagaagg gacccaccag gttgattcac agaggggata agtactctgt cagctggtct 2400
   caaagaggct gaaaccctct gagcttccct cagggccagc ttctaaatcc aggtgccagg 2460
   gatctgctca ggagccccgg ctccaggcct caacctcttg cctgtcattc cttagcatct 2520
   tactttgctt cagggaaaag gtgcaccttc cttagaaccc cagaaaatgc tgaagttcta 2580
   ggagagacag ggtttatact tgtaagcctt tatcagctga tatttcacct gtgaaagcct 2640
   tggggaagag attggttaca cggaacagga tcaggagggg agattactgc tccctctcac 2700
   tgctgggagc agtgcaaact ggtttctgga aggcagttca acaacatgca gcaaagccct 2760
   gcaagtccac acctaggaac ccatcctgag gagcgaatcc tgaagggatg ccaacattga 2820
   cacagctttg tgtgctaccg ggaagcacag caaacaaccc aaacgcccag aagcagaggg 2880
   ctggacacat caatcatggt ctgtccatag gatgaaacat cacgcagtca cttttaaaat 2940
   gacagagaaa aaggcataat aaccgtacag aattattgtg tttttcatta cacacacatg 3000
   cacctgtcta atctattcac ggaaaaagcc tagaaataca aggaaatatc agttgtttta 3060
   ggatggaggc cttatgggtg gtgtttattt attcttttgt acatttctac attttttgtc 3120
   ttctttaatc attttctatc acaaattaat tttccttttt ttcccagaaa atatatcttt 3180
   tgtacttttt attagtacat catagttgtg catattgtgg gggtacatgt gattttttgg 3240
   tgcatgtata caatgtgtat gtttttttaa accttagcaa aaattagcaa aagtagtggg 3300
   ggaggtaggg aggaatgaaa atatttgcag ggccaggcgc gatggctcat ggctgtaatc 3360
   ccagcacttc gggaggctga ggcaggcaga tcacgaggtc aagagataga gaccatccta 3420
   gccaacatgg tgaaaccctg tctctactaa aaatacaaaa attagctggg catggtagtg 3480
   cgcgcctgta gtcccagcta ctcgagaggc tgaggcagga gaggttgaac ccaggaggtg 3540
   gaggttgcag tgagccgaga ctgcgccact gcactccagc ctgggcgaca gagtgagact 3600
   ccatcaaaaa aaaaaaaaga aggaaagaaa gaaagaagaa agaaacaaag aaagaatatt 3660
   tgcataaatt gtgtttgcgt tgacaaggga taggaactga gaatggggaa ggaagatgag 3720
   acacattttc tctgtctgaa tcagatattt agggaggaat taagaacgga gccctgcctc 3780
   tctatgagtg cacttgatga atacgggact gattctggga aagactgcct ttggtcaggt 3840
   tctcaaaaca ccattgtttt gaactatcca gcagcagttt taattcatgg catttgcaat 3900
   tatatttata gcataaattg tctattctct gcagtgtatt ttttagccct cctcccccag 3960
   aaaatgtaat tggaaatatc aactcatgtt gaatgtgttt gctgtacctg tttatgtttt 4020
   attgattatt ttctgggaga agtttggagt tgtgtttggt ttggctctgt gcaacctcat 4080
   ttatttgctc tgtaaagctg ataagcacaa gaagtgaaat gacactcagg cattttaaga 4140
   tgcgagtcat cttcattctg aactaagtga aaacaaaaca gattttctat caacctaaaa 4200
   tcctcctact gggggcagta gatgttttta gaaaattggc tttggctttg attgacaatg 4260
   tttggaagat gtcctataat ttgagagtgt atgtgtgtgt gtctgggtgt gtgtgtgtaa 4320
   atgctggacc tgggaagatg gaagccctca gccaggagaa taagaggaca caataccccc 4380
   aagccttcca tgacctagag attaagggga tttaacgtct taaaacctcc attaagtaga 4440
   cagtagcaag agacatgttg ccctcagtga tatgcaaagg catatctgat gggtcagtat 4500
   ctgccatcct cctcttttgc aagctctgag gttagactca gccatgggtg aaggaattac 4560
   agcatccatt gggagcaggc agagtccagg aaagagattt ttctgttacc tcagatgccc 4620
   atcgcgccag gagcaatttt cccagcctct tgtagaaagc acatattagt atctatctca 4680
   ctgcttttca gaaactgtat cttcaattgc taagtgggga aaaatgcatt tacaatgcta 4740
   gagctgactt taagaaaaca agttaacatc aggccacagc actcccctcc cccgctgctg 4800
   agaccttgtc gctgtgcacc ttgtcccctc cctcacggct tctggctccc gctcccccaa 4860
   tccctggccc cctgtctgcc cctgccatta aagtggcaag cacaggtatc ctcctccctc 4920
   atcacaactt atccatcctg tgtctgctcc cccagggtgg gtaatgcctg aaatgcctta 4980
   aggtaggggt aacgctcatg gcaggtggcc ctggctctcc tggagacagg gcacaggaat 5040
   gggcaaattc ttcaggcttg gtgaggtgga aagcttgctc ctcgggaatc atggccacag 5100
   tgcccttttt tgtttgttaa tcattaaaaa gcatactaca tgaatatata aagttggaaa 5160
   accccacaga agagccaagt gaagtcctgc ttacacactc ctcctgatcc ctactctccc 5220
   gtgctgtgga ccatgcttag tgtttgacgt gtattattat tatttttgtg tgtgtgtgac 5280
   agagtcttgc tctgttgctc aggctggagt gcaatggcat gatctcggct caccgcaacc 5340
   tccgcctccc agattcaagc gattctcctg ccaactagct gggattacag gtgcccgcca 5400
   ccacgcccag ctaattttgt atttttagta gagacaaggc ttcaccatgt tgatcaagct 5460
   ggtctcaaac tcctgacctc aggtgatccg cccacctcag cctcccaaaa tgctgggctg 5520
   ggattacagg cgtgagccac cacgcccggc ctgatgtgta ttattctaga cctctgctgc 5580
   ctaatagaaa tagcacacgc gccacagaca taatttcaaa ctgtgtagta gccacttaaa 5640
   aaggaaaagt aaaaacaaat aagtgaaatt aactttaatt ctataattca tttaacacaa 5700
   tatatctaaa atatgattgt ttcaacatgt agtcaatacc aaacacatac taatgaggta 5760
   ttttatgtct ttctttattg ttctaagtct ttgaaatcct gccacacacc tcgttttcag 5820
   ctctcatgtc acagtgaaca ttagcgtgcc atctcacact cacagcatgt ctccattcac 5880
   cctggccaca tctcaggtgc gcaacagccc caggtagcca gggtctacca tgcgggacag 5940
   ctctgctctt gaatatcggg aagtttgttc ctgtagagct ggatcacatc ggaaatattt 6000
   tactacgctt tttttttcaa ttcataatgg agacgtttac actttagagt tctgtgttag 6060
   tcttgttcat ggttgcatag tccatgattt ggtacgtcgt taattggttt aatcagtccc 6120
   ccaggtagac taacaaaaaa gtggaggttt ccaatttttc acttagctta aaatacactg 6180
   caacaagcat ccatacattg cctatttaca tgcaaaagta tttccaggtg atcgatccat 6240
   ggaagtgaaa gtgctgggtc aaagacaggc atgcaccctt tgctctgaga ggcaagccag 6300
   tactcagcca agccggccac caatttgcct tggggtttgg acgttgttcc cttagaggta 6360
   aaagcaaaga gttgggctgg ataccctcca tagttcctaa atgctcagtt ccatggccca 6420
   aagggtggtg attcctgcca atgcctttgg tggacgtagt cttgggtggg tgggtgggtg 6480
   acagctacct gctcccactg gagggcaggc ctggattggg cagataagca aatcaaccac 6540
   acacaccttg tctagataag cagaaaacac tcagaaaaga ccccaattgt tttatttgta 6600
   aaattgctct ttggctaatt ttcatgaatg tggatcattc attaaacgtt atcatagaaa 6660
   aagagcagag caacaaaggg gcccggcagt ctgattcccg tgctggcagc ctccaccgtc 6720
   acgctgagct gtggtgaatg cctagggttt cacccgccta cccctcactc ctgctggcgt 6780
   gcaccctttc cccaccctgg tggccccagc aaacatcaca gtccctgagg gccaggaggc 6840
   ttgggtcccc agatgcccac acctgacagg cctagtgcct gagcactctg gtgagggact 6900
   tccctggacc atgggagcac tgccagagga ggctcgaggg cagggacctg gatgggtagc 6960
   aaaggacaga gaacatcggg aaaggagagg gtactacctg tgggaaccag gcaggcacaa 7020
   gaggctggcc ctgagcctct gcgcatctct cctgcagaca ctggctgcac ctgcctagcc 7080
   tctgaaaccc agaccaggca ctcaggcaat gaggggagct ccttaacaca acaccttccc 7140
   aactgctgtg cttttcggag tgagctggcc aggtgatgtt ggccaaagca ggaaggggag 7200
   ctgttgaggt gccccaagat ccttgctgga atcctcaggt tcccagacaa tcagggtaga 7260
   cccggtgatg ggagaaggag atggggacac acatggatcc gaagctacaa gattctgctt 7320
   ctctctcctg tttttgttta ttcttttaag atgttttatt gctaaataaa gcattttaaa 7380
   aagtgtataa gatgtctgca catagtttag agaatgataa agcagatcct tgtgttttac 7440
   catggggccc tttcccactg cactccttcc ctcccagagg cagcactgcc tgacttctgc 7500
   ctggctcgtt cctgcctttg ctctttcttt ctctcttttt ccctcccttc ctccctccct 7560
   tccttccttc ttcccttccc ttcgtttctt ctttttcttt ccttccttcc ttctttcttt 7620
   tcttttcttt tcttttctat ttttattttt atttttttga gacagagttt tgctcttgtc 7680
   acctaggctg aagtgcagtg gtgctatctc ggctcactgc aaccttcacc tcccatgttc 7740
   aagcgattct cctacctcag cctcccaaat agttgggatt acaggcaact gccaccacgc 7800
   ctggctaatt tttttgtatt tttagtagcg atggggtttc accacgttgg ccaggctggt 7860
   ctcgaactcc tgacctccag agatccaccc accttggcct cccaaagtgc tgggattaca 7920
   ggtgtgagcc actgcgcctg gcccctggct tttctttatg tttcatttag tttagttttg 7980
   aggtttacat attttttata tttatttatt tatttatttt caaatggagt ctcactctgt 8040
   cgcccaggct ggagtgcagt ggtgtgatct cagctcaccg caacctctgc ctcctgggtt 8100
   caagctattc tcctgcctca gcctcccgag tagctgggat tacaggcgcc tgtcaccatg 8160
   cctggctaat tttgtatttt ttagtagaga cgaggtttca ccatgttggc caggctggtc 8220
   tcgaactcct gacctcaggt gatccacccg cctcggcctc ccaaagtgct gggattacag 8280
   acatgagcca ccatgcccag ccatattttt aaataattta ttgagatgaa atttgtatca 8340
   cataaaatgg accattttca agtgaccaat tcagtggtat ttagtacagt catgaggtca 8400
   tgcaactacc ttgtctctct gtccttactt ccaacactac aaagtaaaac cccctaccca 8460
   ttaagcagtt cctccccatt tcctctccct gtggccttgc ccctggaaac cactagtttg 8520
   tgctttgtct gtatggattt atctattctg gatgtttcat ctaaatggaa tcatacatat 8580
   gtcacctttt atgcctggct tctatcagtt agcatcacgt ttccaaggtt cacccatgtg 8640
   gcatgcttca gaacttcgtt catttatgtg gctaaataat attcttttgt ttgtataaca 8700
   tataccacaa tttgtttatc catctatcca ctgatggaca tgtgagctat ggccaccatt 8760
   tggctgttat gaatagtgct gcaatgctac atgtttattt gagtctctgt tttcatttct 8820
   cttgggtata tacctagcag taaaattgct gggtcatatg gtaattctac atttaactga 8880
   ggaactgcca aatcattttc caaagtgact gtaccatttt acactttcac cagcaatgtc 8940
   tgaaggttcc aattcctcca catccttgca aacactggtt actatttttt tattttggga 9000
   tgtttttaat tataatcatc ctagtaggaa tgaagtggca cctcatcatg gttttgattt 9060
   gcttttccct aatgtctaaa gatgttgggt atcttttcat gtgtttgttg gccatttgta 9120
   tatcttcatt ggagaattgt ctattcaagt cctttgccca gtttttaatc gggttgtctt 9180
   tttgttgttg agttggaaga gttcttttat atattctgga tattagaccc ttgtcatatt 9240
   ttgatttgca aatatcgtat tccattctat aggttgtggg tttttttcct ttcttaataa 9300
   tgtcctttga tgaatttgat gaacagtttt tcattttaat gaagttcaac ttacctgctt 9360
   tttcttttct tgttcatgct tatggtatca tatctataaa tccatcgatc atgaagataa 9420
   tgaaaattca cacatatatt tcttttaaga gttttatggt tttaggtctt acaatttagt 9480
   ctgatccatt ttgagttaat atttatagct agtgtgaagt aggggtccaa attcattctt 9540
   ttgcatgtgg atatccaatt ttccaagcac tattagttga caagactgtc ttttccccca 9600
   ttgaatggtc ttagcacctt tgctaaaaat cacttggaca tagatatatg gacttatttc 9660
   tgaaatctca atcttactcc actggcctgc atgtctgtcc ttatgctagt accatacttc 9720
   tttgattact gacgctttga aattctgaaa tcagaaaata tgagtcctct aactttgttc 9780
   tttttttcaa gattgctttg tcggccatgt gcggggatca tgtttgtaat cccagcactt 9840
   tgggaggctg aggcgggcag atcacttgag gtcaggagtc ggaggccagc ctggccaaca 9900
   tggtgaaacc ctatctctac taaaaataaa aaaaattagc caggcatagt ggcacatgtc 9960
   tgtagtccca gctactccag aggctgaggc acaagaatca cttgaacctg ggaggcagag 10020
   gctgcagtga gccgagatca tgccactgca ctccagcctg ggcaacagag caagactcca 10080
   tctcaaaata aaaaaacaaa aaaaaaaacc agattgctct gtccatttag cccattgcaa 10140
   ttcatgaata caggatgtct ttcagaaccg tgcacaaaaa atatcttcaa caggtggata 10200
   tggatgaatt aaggtgcaaa atgagaacca ttaaatacat acttgcaagc catttctctt 10260
   aaaaggtact agagctaaga tccttaaaga cttgcatcca ccattattgc agatgactat 10320
   tcagatgttg tgggcagaca tattgtttta taaaggtata agaatggact ctgatgatgg 10380
   cttgggaatg tattggtcca atatccactt tgattactga cgctttgaaa ttctgaaatc 10440
   agaaaatatg agtcctctaa ctttgttctt tttttcaaga ttgctttgtc ggccatgtgc 10500
   ggggatcatg tttgtaatcc cagcactttg ggaggctgag gcgggcagat cacttgaggt 10560
   caggagtcgg aggccagcct ggccaacatg gtgaaaccct atctctacta aaaataaaaa 10620
   aaattagcca ggcatagtgg cacatgtctg tagtcccagc tactccagag gctgaggcac 10680
   aagaatcact tgaacctggg aggcagaggc tgcagtgagc cgagatcatg ccactgcact 10740
   ccagcctggg caacagagca agactccatc tcaaaataaa aaaacaaaaa aaaaaaccag 10800
   attgctctgt ccatttagcc cattgcaatt catgaataca ggatgtcttt cagaaccgtg 10860
   cacaaaaaat atcttcaaca ggtggatatg gatgaattaa ggtgcaaaat gagaaccatt 10920
   aaatacatac ttgcaagcca tttctcttaa aaggtactag agctaagatc cttaaagact 10980
   tgcatccacc attattgcag atgactattc agatgttgtg ggcagacata ttgttttata 11040
   aaggtataag aatggactct gatgatggct tgggaatgta ttggtccaat atccact 11097

<210> 2
<211> 11097
<212> DNA
<213> Artificial Sequence

<220>
<223> chemically treated genomic DNA (Homo sapiens)

<400> 2

agtagttggg attataggta tttattatta tggtcggtta ttttttatat ttttagtaga 60
gatgaggttt tattatgttg gttaggttgg tttcgaattt ttgattttag gtgatttatt 120
tgttttagtt ttttaaagtg ttggaattgt aggcgtgagt tgggttaatt taaaattttt 180
tagtagttag ttttgaaaag taaatagaag taggtgaaaa aaattaataa tattttattt 240
aatttaatat gttttaaata ttttaatgtg taatcgatta ttattataag ttttggtaat 300
ttcgtgtata ttttatattc ggagtatatt ttaattttga ttggttttgt ttttagtatt 360
ttatagttac gcgtggtttg tggttattgt gtgagatatt ttagttttta gttgtaggtt 420
atattgtatg gattttgggg aggaggggtg tttttttggg gaaggatggg gtttttattg 480
tttttatttt atttgaattt gaaaaagagt ttatttgttt agtgaagggt ttattgtttg 540
gagagttatt gattggtagt cggtcgggtt ggttattggt gttgattata gagtttttat 600
attttttagt tatggtagtg tcggttatta gtttattttt ttatgtggtt tttttttatt 660
gtttttagat tgattttaga tgtttcggat tgttttgatt gtgtttttag aatttcggtt 720
acgtagtatt tttttttagt tttcggaagg gttttcgttg aagtcgtgat ttttttaggt 780
tttaggagtt aatattagtt taagaaagaa gattcgggag ttaggttagg gaggttgttg 840
cggttattgt gcgtttaggg gttgtgcgcg ttttagtaaa gtacgtggga gttatttatt 900
agattttttg tacgtttatt ttgcggtgat agattttgag agaaacgaat gaaagtaaag 960
atagatttag aaaatggttt ggtatatgtg ttttcgttag aagtttagag attttttgag 1020
ttttaggttg tttgttaggt attgggtggg ggagtgagtt tagtttttag ttttaggatt 1080
ttatatatgg gtagaatttg ataatttggg tatttttatt ttattttatt tgttttttgg 1140
gtaagttatt tttttatatg atttagagtt tttatttgta aaatgggaat atttttaatt 1200
gtttttacgt ttaatttgtt tgtttttttg tgagaagcgg gaggcggata atgtttaaga 1260
gttttagatg ttggggaaga tttagttttt ttgtagagag ggatagttta gggtttgggt 1320
tttttttttt tttttgggtt gagaaagttt atggaaagtt ggaataatta cgtatattgt 1380
tatagtaaat ttaatagagt ttttatcgtg gtggttttta ggtgattgtg gatgttaagt 1440
aggtaagttt ttggtgaagg ggggagagta gggattagaa gtatttagaa ggggttgaga 1500
gttatgtggg gtttatattg tatttgtagt tgggtttatt ttgattttag gtttaattta 1560
gatgaggaag gattagtagt aattagtgtt atgtgtcgtt tttttttagt ttttcggggt 1620
acgaatattg tttagttgat gaggggattt tgaaagaatt attatgttta attgttttaa 1680
ttatgtaaat tttgttgata tttttagtta gggaagggcg gttgggtggg agggggttat 1740
ggcggggtta ttttaaagga aaagttttag tttttttatt ttttttatat tttaaggttg 1800
tttttgtggg attttatata gaatagtttg gaagtttggg gttttggttt ttttttttgg 1860
tttgggagtt aggttatggg gttatcgttt tatagtaatt atgagggttt aggtttaagt 1920
gtttatatgt tttttatggg gattgttttt tttaaagggt gggttttttt tatttagttt 1980
tttgttttgg ttaaggagga ggttgaaaga gtttgagttg tgtttttttt attttattgt 2040
tgtggtaggt aagtttagat gcgggtaagt ataggtgaag ggaggttagg tgtaggtatt 2100
tttaggtgtg ggtaaattta agcgtggata aatttaggtg tagataatta tagatgtagt 2160
taagtttagg tgttggtaat tttaggtgta ggtaggttta ggtgtaagta agagtaggtg 2220
taggtaagtt taggtgtgtt ttttggggtt tgttatttat ttttttttga ggttggttgg 2280
ataggatttt atggagtagt tggtgaattt gtgtttagtt tgggttttaa gaggttgttt 2340
tagtagaagg gatttattag gttgatttat agaggggata agtattttgt tagttggttt 2400
taaagaggtt gaaatttttt gagttttttt tagggttagt ttttaaattt aggtgttagg 2460

gatttgttta ggagtttcgg ttttaggttt taattttttg tttgttattt tttagtattt 2520
tattttgttt tagggaaaag gtgtattttt tttagaattt tagaaaatgt tgaagtttta 2580
ggagagatag ggtttatatt tgtaagtttt tattagttga tattttattt gtgaaagttt 2640
tggggaagag attggttata cggaatagga ttaggagggg agattattgt ttttttttat 2700
tgttgggagt agtgtaaatt ggtttttgga aggtagttta ataatatgta gtaaagtttt 2760
gtaagtttat atttaggaat ttattttgag gagcgaattt tgaaggatgg ttaatattga 2820
tatagttttg tgtgttatcg ggaagtatag taaataattt aaacgtttag aagtagaggg 2880
ttggatatat taattatggt ttgtttatag gatgaaatat tacgtagtta tttttaaaat 2940
gatagagaaa aaggtataat aatcgtatag aattattgtg ttttttatta tatatatatg 3000
tatttgttta atttatttac ggaaaaagtt tagaaatata aggaaatatt agttgtttta 3060
ggatggaggt tttatgggtg gtgtttattt atttttttgt atatttttat attttttgtt 3120
ttttttaatt attttttatt ataaattaat tttttttttt tttttagaaa atatattttt 3180
tgtatttttt attagtatat tatagttgtg tatattgtgg gggtatatgt gattttttgg 3240
tgtatgtata taatgtgtat gtttttttaa attttagtaa aaattagtaa aagtagtggg 3300
ggaggtaggg aggaatgaaa atatttgtag ggttaggcgc gatggtttat ggttgtaatt 3360
ttagtatttc gggaggttga ggtaggtaga ttacgaggtt aagagataga gattatttta 3420
gttaatatgg tgaaattttg tttttattaa aaatataaaa attagttggg tatggtagtg 3480
cgcgtttgta gttttagtta ttcgagaggt tgaggtagga gaggttgaat ttaggaggtg 3540
gaggttgtag tgagtcgaga ttgcgttatt gtattttagt ttgggcgata gagtgagatt 3600
ttattaaaaa aaaaaaaaga aggaaagaaa gaaagaagaa agaaataaag aaagaatatt 3660
tgtataaatt gtgtttgcgt tgataaggga taggaattga gaatggggaa ggaagatgag 3720
atatattttt tttgtttgaa ttagatattt agggaggaat taagaacgga gttttgtttt 3780
tttatgagtg tatttgatga atacgggatt gattttggga aagattgttt ttggttaggt 3840
ttttaaaata ttattgtttt gaattattta gtagtagttt taatttatgg tatttgtaat 3900
tatatttata gtataaattg tttatttttt gtagtgtatt ttttagtttt tttttttttag 3960
aaaatgtaat tggaaatatt aatttatgtt gaatgtgttt gttgtatttg tttatgtttt 4020
attgattatt ttttgggaga agtttggagt tgtgtttggt ttggttttgt gtaattttat 4080
ttatttgttt tgtaaagttg ataagtataa gaagtgaaat gatatttagg tattttaaga 4140
tgcgagttat ttttattttg aattaagtga aaataaaata gattttttat taatttaaaa 4200
tttttttatt gggggtagta gatgttttta gaaaattggt tttggttttg attgataatg 4260
tttggaagat gttttataat ttgagagtgt atgtgtgtgt gtttgggtgt gtgtgtgtaa 4320
atgttggatt tgggaagatg gaagttttta gttaggagaa taagaggata taatattttt 4380
aagtttttta tgatttagag attaagggga tttaacgttt taaaattttt attaagtaga 4440
tagtagtaag agatatgttg tttttagtga tatgtaaagg tatatttgat gggttagtat 4500
ttgttatttt ttttttttgt aagttttgag gttagattta gttatgggtg aaggaattat 4560
agtatttatt gggagtaggt agagtttagg aaagagattt ttttgttatt ttagatgttt 4620
atcgcgttag gagtaatttt tttagttttt tgtagaaagt atatattagt atttatttta 4680
ttgtttttta gaaattgtat ttttaattgt taagtgggga aaaatgtatt tataatgtta 4740
gagttgattt taagaaaata agttaatatt aggttatagt attttttttt ttcgttgttg 4800
agattttgtc gttgtgtatt ttgttttttt ttttacggtt tttggttttc gtttttttaa 4860
tttttggttt tttgtttgtt tttgttatta aagtggtaag tataggtatt tttttttttt 4920
attataattt atttattttg tgtttgtttt tttagggtgg gtaatgtttg aaatgtttta 4980
aggtaggggt aacgtttatg gtaggtggtt ttggtttttt tggagatagg gtataggaat 5040
gggtaaattt tttaggtttg gtgaggtgga aagtttgttt ttcgggaatt atggttatag 5100
tgtttttttt tgtttgttaa ttattaaaaa gtatattata tgaatatata aagttggaaa 5160
attttataga agagttaagt gaagttttgt ttatatattt tttttgattt ttattttttc 5220
gtgttgtgga ttatgtttag tgtttgacgt gtattattat tatttttgtg tgtgtgtgat 5280
agagttttgt tttgttgttt aggttggagt gtaatggtat gatttcggtt tatcgtaatt 5340
ttcgtttttt agatttaagc gatttttttg ttaattagtt gggattatag gtgttcgtta 5400
ttacgtttag ttaattttgt atttttagta gagataaggt tttattatgt tgattaagtt 5460
ggttttaaat ttttgatttt aggtgattcg tttattttag ttttttaaaa tgttgggttg 5520
ggattatagg cgtgagttat tacgttcggt ttgatgtgta ttattttaga tttttgttgt 5580
ttaatagaaa tagtatacgc gttatagata taattttaaa ttgtgtagta gttatttaaa 5640
aaggaaaagt aaaaataaat aagtgaaatt aattttaatt ttataattta tttaatataa 5700
tatatttaaa atatgattgt tttaatatgt agttaatatt aaatatatat taatgaggta 5760
ttttatgttt ttttttattg ttttaagttt ttgaaatttt gttatatatt tcgtttttag 5820
tttttatgtt atagtgaata ttagcgtgtt attttatatt tatagtatgt ttttatttat 5880
tttggttata ttttaggtgc gtaatagttt taggtagtta gggtttatta tgcgggatag 5940
ttttgttttt gaatatcggg aagtttgttt ttgtagagtt ggattatatc ggaaatattt 6000
tattacgttt ttttttttaa tttataatgg agacgtttat attttagagt tttgtgttag 6060
ttttgtttat ggttgtatag tttatgattt ggtacgtcgt taattggttt aattagtttt 6120
ttaggtagat taataaaaaa gtggaggttt ttaatttttt atttagttta aaatatattg 6180
taataagtat ttatatattg tttatttata tgtaaaagta tttttaggtg atcgatttat 6240

ggaagtgaaa gtgttgggtt aaagataggt atgtattttt tgttttgaga ggtaagttag 6300
tatttagtta agtcggttat taatttgttt tggggtttgg acgttgtttt tttagaggta 6360
aaagtaaaga gttgggttgg atatttttta tagtttttaa atgtttagtt ttatggttta 6420
aagggtggtg attttgttta atgttttggt tggacgtagt tttgggtggg tgggtgggtg 6480
atagttattt gtttttattg gagggtaggt ttggattggg tagataagta aattaattat 6540
atatattttg tttagataag tagaaaatat ttagaaaaga ttttaattgt tttatttgta 6600
aaattgtttt ttggttaatt tttatgaatg tggattattt attaaacgtt attatagaaa 6660
aagagtagag taataaaggg gttcggtagt ttgattttcg tgttggtagt ttttatcgtt 6720
acgttgagtt gtggtgaatg tttagggttt tattcgttta ttttttattt ttgttggcgt 6780
gtattttttt tttattttgg tggttttagt aaatattata gtttttgagg gttaggaggt 6840
ttgggttttt agatgtttat atttgatagg tttagtgttt gagtattttg gtgagggatt 6900
tttttggatt atgggagtat tgttagagga ggttcgaggg tagggatttg gatgggtagt 6960
aaaggataga gaatatcggg aaaggagagg gtattatttg tgggaattag gtaggtataa 7020
gaggttggtt ttgagttttt gcgtattttt tttgtagata ttggttgtat ttgtttagtt 7080
tttgaaattt agattaggta tttaggtaat gaggggagtt ttttaatata atattttttt 7140
aattgttgtg tttttcggag tgagttggtt aggtgatgtt ggttaaagta ggaaggggag 7200
ttgttgaggt gttttaagat ttttgttgga atttttaggt ttttagataa ttagggtaga 7260
ttcggtgatg ggagaaggag atggggatat atatggattc gaagttataa gattttgttt 7320
tttttttttg tttttgttta tttttttaag atgttttatt gttaaataaa gtattttaaa 7380
aagtgtataa gatgtttgta tatagtttag agaatgataa agtagatttt tgtgttttat 7440
tatggggttt ttttttattg tatttttttt tttttagagg tagtattgtt tgatttttgt 7500
ttggttcgtt tttgtttttg tttttttttt tttttttttt tttttttttt tttttttttt 7560
tttttttttt tttttttttt ttcgtttttt tttttttttt tttttttttt tttttttttt 7620
tttttttttt ttttttttat ttttattttt atttttttga gatagagttt tgtttttgtt 7680
atttaggttg aagtgtagtg gtgttatttc ggtttattgt aatttttatt ttttatgttt 7740
aagcgatttt tttattttag ttttttaaat agttgggatt ataggtaatt gttattacgt 7800
ttggttaatt tttttgtatt tttagtagcg atggggtttt attacgttgg ttaggttggt 7860
ttcgaatttt tgatttttag agatttattt attttggttt tttaaagtgt tgggattata 7920
ggtgtgagtt attgcgtttg gtttttggtt tttttttatg ttttatttag tttagttttg 7980
aggtttatat attttttata tttatttatt tatttatttt taaatggagt tttattttgt 8040
cgtttaggtt ggagtgtagt ggtgtgattt tagtttatcg taatttttgt tttttgggtt 8100
taagttattt ttttgtttta gtttttcgag tagttgggat tataggcgtt tgttattatg 8160
tttggttaat ttttgtattt ttagtagaga cgaggtttta ttatgttggt taggttggtt 8220
tcgaattttt gattttaggt gatttattcg tttcggtttt ttaaagtgtt gggattatag 8280
atatgagtta ttatgtttag ttatattttt aaataattta ttgagatgaa atttgtatta 8340
tataaaatgg attattttta agtgattaat ttagtggtat ttagtatagt tatgaggtta 8400
tgtaattatt ttgttttttt gtttttattt ttaatattat aaagtaaaat tttttattta 8460
ttaagtagtt tttttttatt tttttttttt gtggttttgt ttttggaaat tattagtttg 8520
tgttttgttt gtatggattt atttattttg gatgttttat ttaaatggaa ttatatatat 8580
gttatttttt atgtttggtt tttattagtt agtattacgt ttttaaggtt tatttatgtg 8640
gtatgtttta gaatttcgtt tatttatgtg gttaaataat atttttttgt ttgtataata 8700
tatattataa tttgtttatt tatttattta ttgatggata tgtgagttat ggttattatt 8760
tggttgttat gaatagtgtt gtaatgttat atgtttattt gagtttttgt ttttattttt 8820
tttgggtata tatttagtag taaaattgtt gggttatatg gtaattttat atttaattga 8880
ggaattgtta aattattttt taaagtgatt gtattatttt atatttttat tagtaatgtt 8940
tgaaggtttt aattttttta tatttttgta aatattggtt attatttttt tattttggga 9000
tgtttttaat tataattatt ttagtaggaa tgaagtggta ttttattatg gttttgattt 9060
gttttttttt aatgtttaaa gatgttgggt atttttttat gtgtttgttg gttatttgta 9120
tatttttatt ggagaattgt ttatttaagt tttttgttta gtttttaatc gggttgtttt 9180
tttgttgttg agttggaaga gtttttttat atattttgga tattagattt ttgttatatt 9240
ttgatttgta aatatcgtat tttattttat aggttgtggg tttttttttt tttttaataa 9300
tgttttttga tgaatttgat gaatagtttt ttattttaat gaagtttaat ttatttgttt 9360
tttttttttt tgtttatgtt tatggtatta tatttataaa tttatcgatt atgaagataa 9420
tgaaaattta tatatatatt ttttttaaga gttttatggt tttaggtttt ataatttagt 9480
ttgatttatt ttgagttaat atttatagtt agtgtgaagt aggggtttaa atttattttt 9540
ttgtatgtgg atatttaatt ttttaagtat tattagttga taagattgtt ttttttttta 9600
ttgaatggtt ttagtatttt tgttaaaaat tatttggata tagatatatg gatttatttt 9660
tgaaatttta attttatttt attggtttgt atgtttgttt ttatgttagt attatatttt 9720
tttgattatt gacgttttga aattttgaaa ttagaaaata tgagtttttt aattttgttt 9780
ttttttttaa gattgttttg tcggttatgt gcggggatta tgtttgtaat tttagtattt 9840
tgggaggttg aggcgggtag attatttgag gttaggagtc ggaggttagt ttggttaata 9900
tggtgaaatt ttatttttat taaaaataaa aaaaattagt taggtatagt ggtatatgtt 9960
tgtagtttta gttattttag aggttgaggt ataagaatta tttgaatttg ggaggtagag 10020

gttgtagtga gtcgagatta tgttattgta ttttagtttg ggtaatagag taagatttta 10080
ttttaaaata aaaaaataaa aaaaaaaatt agattgtttt gtttatttag tttattgtaa 10140
tttatgaata taggatgttt tttagaatcg tgtataaaaa atatttttaa taggtggata 10200
tggatgaatt aaggtgtaaa atgagaatta ttaaatatat atttgtaagt tatttttttt 10260
aaaaggtatt agagttaaga tttttaaaga tttgtattta ttattattgt agatgattat 10320
ttagatgttg tgggtagata tattgtttta taaaggtata agaatggatt ttgatgatgg 10380
tttgggaatg tattggttta atatttattt tgattattga cgttttgaaa ttttgaaatt 10440
agaaaatatg agttttttaa ttttgttttt ttttttaaga ttgttttgtc ggttatgtgc 10500
ggggattatg tttgtaattt tagtattttg ggaggttgag gcgggtagat tatttgaggt 10560
taggagtcgg aggttagttt ggttaatatg gtgaaatttt atttttatta aaaataaaaa 10620
aaattagtta ggtatagtgg tatatgtttg tagttttagt tattttagag gttgaggtat 10680
aagaattatt tgaatttggg aggtagaggt tgtagtgagt cgagattatg ttattgtatt 10740
ttagtttggg taatagagta agatttattt ttaaaataaa aaaataaaaa aaaaaattag 10800
attgttttgt ttatttagtt tattgtaatt tatgaatata ggatgttttt tagaatcgtg 10860
tataaaaaat atttttaata ggtggatatg gatgaattaa ggtgtaaaat gagaattatt 10920
aaatatatat ttgtaagtta ttttttttaa aaggtattag agttaagatt tttaaagatt 10980
tgtatttatt attattgtag atgattattt agatgttgtg ggtagatata ttgttttata 11040
aaggtataag aatggatttt gatgatggtt tgggaatgta ttggtttaat atttatt 11097

<210> 3
<211> 11097
<212> DNA
<213> Artificial Sequence

<220>
<223> chemically treated genomic DNA (Homo sapiens)

<400> 3

agtggatatt ggattaatat atttttaagt tattattaga gtttattttt atatttttat 60
aaaataatat gtttgtttat aatatttgaa tagttatttg taataatggt ggatgtaagt 120
ttttaaggat tttagtttta gtatttttta agagaaatgg tttgtaagta tgtatttaat 180
ggtttttatt ttgtatttta atttatttat atttatttgt tgaagatatt ttttgtgtac 240
ggttttgaaa gatattttgt atttatgaat tgtaatgggt taaatggata gagtaatttg 300
gttttttttt ttgttttttt attttgagat ggagttttgt tttgttgttt aggttggagt 360
gtagtggtat gatttcggtt tattgtagtt tttgtttttt aggtttaagt gatttttgtg 420
ttttagtttt tggagtagtt gggattatag atatgtgtta ttatgtttgg ttaatttttt 480
ttatttttag tagagatagg gttttattat gttggttagg ttggttttcg atttttgatt 540
ttaagtgatt tgttcgtttt agttttttaa agtgttggga ttataaatat gattttcgta 600
tatggtcgat aaagtaattt tgaaaaaaag aataaagtta gaggatttat attttttgat 660
tttagaattt taaagcgtta gtaattaaag tggatattgg attaatatat ttttaagtta 720
ttattagagt ttatttttat atttttataa aataatatgt ttgtttataa tatttgaata 780
gttatttgta ataatggtgg atgtaagttt ttaaggattt tagttttagt attttttaag 840
agaaatggtt tgtaagtatg tatttaatgg tttttatttt gtattttaat ttatttatat 900
ttatttgttg aagatatttt ttgtgtacgg ttttgaaaga tattttgtat ttatgaattg 960
taatgggtta aatggataga gtaatttggt tttttttttt gtttttttat tttgagatgg 1020
agttttgttt tgttgtttag gttggagtgt agtggtatga tttcggttta ttgtagtttt 1080
tgttttttag gtttaagtga tttttgtgtt ttagtttttg gagtagttgg gattatagat 1140
atgtgttatt atgtttggtt aatttttttt atttttagta gagatagggt tttattatgt 1200
tggttaggtt ggttttcgat ttttgatttt aagtgatttg ttcgttttag ttttttaaag 1260
tgttgggatt ataaatatga ttttcgtata tggtcgataa agtaattttg aaaaaaagaa 1320
taaagttaga ggatttatat tttttgattt tagaatttta aagcgttagt aattaaagaa 1380
gtatggtatt agtataagga tagatatgta ggttagtgga gtaagattga gattttagaa 1440
ataagtttat atatttatgt ttaagtgatt tttagtaaag gtgttaagat tatttaatgg 1500
gggaaaagat agttttgtta attaatagtg tttggaaaat tggatattta tatgtaaaag 1560
aatgaatttg gatttttatt ttatattagt tataaatatt aatttaaaat ggattagatt 1620
aaattgtaag atttaaaatt ataaaatttt taaaagaaat atatgtgtga atttttatta 1680
tttttatgat cgatggattt atagatatga tattataagt atgaataaga aaagaaaaag 1740
taggtaagtt gaattttatt aaaatgaaaa attgtttatt aaatttatta aaggatatta 1800
ttaagaaagg aaaaaaattt ataatttata gaatggaata cgatatttgt aaattaaaat 1860
atgataaggg tttaatattt agaatatata aaagaatttt tttaatttaa taataaaaag 1920
ataattcgat taaaaattgg gtaaaggatt tgaatagata attttttaat gaagatatat 1980
aaatggttaa taaatatatg aaaagatatt taatattttt agatattagg gaaaagtaaa 2040

ttaaaattat gatgaggtgt tattttattt ttattaggat gattataatt aaaaatattt 2100
taaaataaaa aaatagtaat tagtgtttgt aaggatgtgg aggaattgga atttttagat 2160
attgttggtg aaagtgtaaa atggtatagt tattttggaa aatgatttgg tagtttttta 2220
gttaaatgta gaattattat atgatttagt aattttattg ttaggtatat atttaagaga 2280
aatgaaaata gagatttaaa taaatatgta gtattgtagt attatttata atagttaaat 2340
ggtggttata gtttatatgt ttattagtgg atagatggat aaataaattg tggtatatgt 2400
tatataaata aaagaatatt atttagttat ataaatgaac gaagttttga agtatgttat 2460
atgggtgaat tttggaaacg tgatgttaat tgatagaagt taggtataaa aggtgatata 2520
tgtatgattt tatttagatg aaatatttag aatagataaa tttatataga taaagtataa 2580
attagtggtt tttaggggta aggttatagg gagaggaaat ggggaggaat tgtttaatgg 2640
gtagggggtt ttattttgta gtgttggaag taaggataga gagataaggt agttgtatga 2700
ttttatgatt gtattaaata ttattgaatt ggttatttga aaatggttta ttttatgtga 2760
tataaatttt attttaataa attatttaaa aatatggttg ggtatggtgg tttatgtttg 2820
taattttagt attttgggag gtcgaggcgg gtggattatt tgaggttagg agttcgagat 2880
tagtttggtt aatatggtga aatttcgttt ttattaaaaa tataaaaatt agttaggtat 2940
ggtgataggc gtttgtaatt ttagttattc gggaggttga ggtaggagaa tagtttgaat 3000
ttaggaggta gaggttgcgg tgagttgaga ttatattatt gtattttagt ttgggcgata 3060
gagtgagatt ttatttgaaa ataaataaat aaataaatat aaaaaatatg taaattttaa 3120
aattaaatta aatgaaatat aaagaaaagt taggggttag gcgtagtggt ttatatttgt 3180
aattttagta ttttgggagg ttaaggtggg tggatttttg gaggttagga gttcgagatt 3240
agtttggtta acgtggtgaa attttatcgt tattaaaaat ataaaaaaat tagttaggcg 3300
tggtggtagt tgtttgtaat tttaattatt tgggaggttg aggtaggaga atcgtttgaa 3360
tatgggaggt gaaggttgta gtgagtcgag atagtattat tgtattttag tttaggtgat 3420
aagagtaaaa ttttgtttta aaaaaataaa aataaaaata gaaaagaaaa gaaaagaaaa 3480
gaaagaagga aggaaggaaa gaaaaagaag aaacgaaggg aagggaagaa ggaaggaagg 3540
gagggaggaa gggagggaaa aagagagaaa gaaagagtaa aggtaggaac gagttaggta 3600
gaagttaggt agtgttgttt ttgggaggga aggagtgtag tgggaaaggg ttttatggta 3660
aaatataagg atttgtttta ttatttttta aattatgtgt agatatttta tatatttttt 3720
aaaatgtttt atttagtaat aaaatatttt aaaagaataa ataaaaatag gagagagaag 3780
tagaattttg tagtttcgga tttatgtgtg tttttatttt ttttttttat tatcgggttt 3840
attttgattg tttgggaatt tgaggatttt agtaaggatt tggggtattt ttaatagttt 3900
ttttttttgt tttggttaat attatttggt tagtttattt cgaaaagtat agtagttggg 3960
aaggtgttgt gttaaggagt ttttttattt gtttgagtgt ttggtttggg ttttagaggt 4020
taggtaggtg tagttagtgt ttgtaggaga gatgcgtaga ggtttagggt tagttttttg 4080
tgtttgtttg gtttttatag gtagtatttt tttttttttc gatgtttttt gttttttgtt 4140
atttatttag gtttttgttt tcgagttttt tttggtagtg tttttatggt ttagggaagt 4200
tttttattag agtgtttagg tattaggttt gttaggtgtg ggtatttggg gatttaagtt 4260
ttttggtttt tagggattgt gatgtttgtt ggggttatta gggtggggaa agggtgtacg 4320
ttagtaggag tgaggggtag gcgggtgaaa ttttaggtat ttattatagt ttagcgtgac 4380
ggtggaggtt gttagtacgg gaattagatt gtcgggtttt tttgttgttt tgtttttttt 4440
ttatgataac gtttaatgaa tgatttatat ttatgaaaat tagttaaaga gtaattttat 4500
aaataaaata attggggttt tttttgagtg ttttttgttt atttagataa ggtgtgtgtg 4560
gttgatttgt ttatttgttt aatttaggtt tgttttttag tgggagtagg tagttgttat 4620
ttatttattt atttaagatt acgtttatta aaggtattgg taggaattat tattttttgg 4680
gttatggaat tgagtattta ggaattatgg agggtattta gtttaatttt ttgtttttat 4740
ttttaaggga ataacgttta aattttaagg taaattggtg gtcggtttgg ttgagtattg 4800
gtttgttttt tagagtaaag ggtgtatgtt tgtttttgat ttagtatttt tatttttatg 4860
gatcgattat ttggaaatat ttttgtatgt aaataggtaa tgtatggatg tttgttgtag 4920
tgtattttaa gttaagtgaa aaattggaaa tttttatttt tttgttagtt tatttggggg 4980
attgattaaa ttaattaacg acgtattaaa ttatggatta tgtaattatg aataagatta 5040
atatagaatt ttaaagtgta aacgttttta ttatgaattg aaaaaaaaag cgtagtaaaa 5100
tattttcgat gtgatttagt tttataggaa taaatttttc gatatttaag agtagagttg 5160
tttcgtatgg tagattttgg ttatttgggg ttgttgcgta tttgagatgt ggttagggtg 5220
aatggagata tgttgtgagt gtgagatggt acgttaatgt ttattgtgat atgagagttg 5280
aaaacgaggt gtgtggtagg attttaaaga tttagaataa taaagaaaga tataaaatat 5340
tttattagta tgtgtttggt attgattata tgttgaaata attatatttt agatatattg 5400
tgttaaatga attatagaat taaagttaat tttatttatt tgtttttatt tttttttttt 5460
aagtggttat tatatagttt gaaattatgt ttgtggcgcg tgtgttattt ttattaggta 5520
gtagaggttt agaataatat atattaggtc gggcgtggtg gtttacgttt gtaattttag 5580
tttagtattt tgggaggttg aggtgggcgg attatttgag gttaggagtt tgagattagt 5640
ttgattaata tggtgaagtt ttgtttttat taaaaatata aaattagttg ggcgtggtgg 5700
cgggtatttg taattttagt tagttggtag gagaatcgtt tgaatttggg aggcggaggt 5760
tgcggtgagt cgagattatg ttattgtatt ttagtttgag taatagagta agattttgtt 5820

atatatatat aaaaataata ataatatacg ttaaatatta agtatggttt atagtacggg 5880
agagtaggga ttaggaggag tgtgtaagta ggattttatt tggttttttt gtggggtttt 5940
ttaattttat atatttatgt agtatgtttt ttaatgatta ataaataaaa aagggtattg 6000
tggttatgat tttcgaggag taagtttttt attttattaa gtttgaagaa tttgtttatt 6060
tttgtgtttt gtttttagga gagttagggt tatttgttat gagcgttatt tttattttaa 6120
ggtattttag gtattattta ttttggggga gtagatatag gatggataag ttgtgatgag 6180
ggaggaggat atttgtgttt gttattttaa tggtaggggt agataggggg ttagggattg 6240
ggggagcggg agttagaagt cgtgagggag gggataaggt gtatagcgat aaggttttag 6300
tagcggggga ggggagtgtt gtggtttgat gttaatttgt ttttttaaag ttagttttag 6360
tattgtaaat gtattttttt ttatttagta attgaagata tagtttttga aaagtagtga 6420
gatagatatt aatatgtgtt ttttataaga ggttgggaaa attgtttttg gcgcgatggg 6480
tatttgaggt aatagaaaaa tttttttttt ggattttgtt tgtttttaat ggatgttgta 6540
atttttttat ttatggttga gtttaatttt agagtttgta aaagaggagg atggtagata 6600
ttgatttatt agatatgttt ttgtatatta ttgagggtaa tatgtttttt gttattgttt 6660
atttaatgga ggttttaaga cgttaaattt ttttaatttt taggttatgg aaggtttggg 6720
ggtattgtgt ttttttattt ttttggttga gggtttttat ttttttaggt ttagtattta 6780
tatatatata tttagatata tatatatata tttttaaatt ataggatatt ttttaaatat 6840
tgttaattaa agttaaagtt aattttttaa aaatatttat tgtttttagt aggaggattt 6900
taggttgata gaaaatttgt tttgttttta tttagtttag aatgaagatg attcgtattt 6960
taaaatgttt gagtgttatt ttattttttg tgtttattag ttttatagag taaataaatg 7020
aggttgtata gagttaaatt aaatataatt ttaaattttt tttagaaaat aattaataaa 7080
atataaatag gtatagtaaa tatatttaat atgagttgat atttttaatt atattttttg 7140
ggggaggagg gttaaaaaat atattgtaga gaatagataa tttatgttat aaatataatt 7200
gtaaatgtta tgaattaaaa ttgttgttgg atagtttaaa ataatggtgt tttgagaatt 7260
tgattaaagg tagttttttt tagaattagt ttcgtattta ttaagtgtat ttatagagag 7320
gtagggtttc gtttttaatt tttttttaaa tatttgattt agatagagaa aatgtgtttt 7380
attttttttt tttattttta gttttatttt tttgttaacg taaatataat ttatgtaaat 7440
attttttttt tgtttttttt tttttttttt tttttttttt tttttttttt ttgatggagt 7500
tttattttgt cgtttaggtt ggagtgtagt ggcgtagttt cggtttattg taatttttat 7560
tttttgggtt taattttttt tgttttagtt tttcgagtag ttgggattat aggcgcgtat 7620
tattatgttt agttaatttt tgtattttta gtagagatag ggttttatta tgttggttag 7680
gatggttttt attttttgat ttcgtgattt gtttgtttta gtttttcgaa gtgttgggat 7740
tatagttatg agttatcgcg tttggttttg taaatatttt tatttttttt tatttttttt 7800
attatttttg ttaatttttg ttaaggttta aaaaaatata tatattgtat atatgtatta 7860
aaaaattata tgtattttta taatatgtat aattatgatg tattaataaa aagtataaaa 7920
gatatatttt ttgggaaaaa aaggaaaatt aatttgtgat agaaaatgat taaagaagat 7980
aaaaaatgta gaaatgtata aaagaataaa taaatattat ttataaggtt tttattttaa 8040
aataattgat atttttttgt atttttaggt ttttttcgtg aatagattag ataggtgtat 8100
gtgtgtgtaa tgaaaaatat aataattttg tacggttatt atgttttttt ttttgttatt 8160
ttaaaagtga ttgcgtgatg ttttatttta tggatagatt atgattgatg tgtttagttt 8220
tttgtttttg ggcgtttggg ttgtttgttg tgtttttcgg tagtatataa agttgtgtta 8280
atgttggtat ttttttagga ttcgtttttt aggatgggtt tttaggtgtg gatttgtagg 8340
gttttgttgt atgttgttga attgtttttt agaaattagt ttgtattgtt tttagtagtg 8400
agagggagta gtaatttttt tttttgattt tgtttcgtgt aattaatttt ttttttaagg 8460
tttttatagg tgaaatatta gttgataaag gtttataagt ataaattttg ttttttttag 8520
aattttagta ttttttgggg ttttaaggaa ggtgtatttt ttttttgaag taaagtaaga 8580
tgttaaggaa tgataggtaa gaggttgagg tttggagtcg ggtttttttga gtagattttt 8640
ggtatttgga tttagaagtt ggttttgagg gaagtttaga gggttttagt ttttttgaga 8700
ttagttgata gagtatttat tttttttgtg aattaatttg gtgggttttt tttgttgaga 8760
tagttttttg gaatttaggt tgaatatagg tttattagtt gttttatagg gttttgttta 8820
gttagtttta agaggagatg agtaataggt tttagaaagt atatttgagt ttgtttgtat 8880
ttgtttttat ttgtatttga gtttatttgt atttgaggtt attaatattt gagtttaatt 8940
gtatttgtgg ttatttgtat ttgagtttgt ttacgtttga gtttatttat atttgaggat 9000
atttgtattt gatttttttt tatttgtgtt tattcgtatt tgagtttatt tgttatagta 9060
gtggaatgga gagggtatag tttaggtttt tttagttttt tttttggtta gggtagggag 9120
ttgggtgagg agggtttatt ttttaagagg agtagttttt atgaggagta tgtgagtatt 9180
tgggtttggg tttttatgat tgttgtgaag cgatggtttt atgatttgat ttttaggtta 9240
gaaaaggaag ttagggtttt aagtttttag gttgttttgt gtggaatttt ataaaggtag 9300
ttttaggatg tgagagaggt aggggagtta gagttttttt tttgaagtgg tttcgttatg 9360
gttttttttt atttagtcgt ttttttttgg ttggaaatgt tagtagggtt tgtataattg 9420
agataattgg atataatggt ttttttagaa tttttttatt agttgggtag tgttcgtgtt 9480
tcggggagtt gggagaaggc ggtatatggt attaattatt gttaattttt ttttatttaa 9540
gttgggtttg aggttagggt gaatttagtt gtaaatgtag tgtgagtttt atatgatttt 9600

tagttttttt tgagtgtttt taatttttgt tttttttttt ttattaggag tttgtttgtt 9660
tggtatttat agttatttga aaattattac ggtgggagtt ttgttggatt tgttgtaata 9720
gtatgcgtgg ttattttagt tttttatgag tttttttagt ttaggaggaa agaaaaaatt 9780
taaattttga attatttttt tttgtagagg ggttgggttt tttttagtat ttgaggtttt 9840
tgggtattgt tcgtttttcg ttttttatag aggagtagat agattggacg taagggtaat 9900
taggggtgtt tttattttat agatgaggat tttgagttat gtgagagggt ggtttgttta 9960
gagagtaggt ggggtggagt gggagtgttt aggttgttaa gttttgttta tatgtagagt 10020
tttgaagttg gaggttgggt ttattttttt atttaatgtt tggtagatag tttggggttt 10080
agggaatttt tgaatttttg acggaagtat atgtgttagg ttattttttg ggtttgtttt 10140
tgtttttatt cgtttttttt agggtttgtt atcgtagagt gggcgtgtaa agggtttggt 10200
gagtggtttt tacgtgtttt gttggagcgc gtatagtttt tgggcgtata atgatcgtag 10260
tagttttttt ggtttggttt tcgagttttt ttttttgaat tggtgttggt ttttgaggtt 10320
tgagagagtt acggttttag cgggaatttt ttcgggggtt gagggagggt gttacgtggt 10380
cggaattttg aaggtatagt tagggtaatt cggggtattt gaaattagtt tgggagtagt 10440
ggggaaagat tatatgggga agtgagttga tggtcggtat tgttatggtt gggaggtgtg 10500
gaggttttgt ggttagtatt aatggttagt tcgatcggtt gttagttagt ggttttttag 10560
gtagtgggtt ttttattaga taaataagtt tttttttagg tttagataag atggaaataa 10620
tgggagtttt attttttttt aagagaatat tttttttttt tagggtttat gtagtgtggt 10680
ttgtaattgg gagttgagat gttttatata gtggttatag gttacgcgtg gttatggagt 10740
gttggaaata gagttagtta gaattgagat atgtttcgaa tgtaaaatat atacgggatt 10800
gttaagattt gtgataataa tcgattgtat attgaaatat ttagaatatg ttgggttgaa 10860
tagaatgtta ttaatttttt ttatttgttt ttatttattt tttaaaattg attattagaa 10920
aattttaaat tggtttagtt tacgtttgta attttagtat tttgggaggt tgaggtaggt 10980
ggattatttg aggttaggag ttcgagatta gtttggttaa tatggtgaaa ttttattttt 11040
attaaaaata taaaaaataa tcggttatgg tggtgggtgt ttgtaatttt agttatt 11097

<210> 4
<211> 11097
<212> DNA
<213> Artificial Sequence

<220>
<223> chemically treated genomic DNA (Homo sapiens)

<400> 4

agtagttggg attataggta tttattatta tggttggtta ttttttatat ttttagtaga 60
gatgaggttt tattatgttg gttaggttgg ttttgaattt ttgattttag gtgatttatt 120
tgttttagtt ttttaaagtg ttggaattgt aggtgtgagt tgggttaatt taaaattttt 180
tagtagttag ttttgaaaag taaatagaag taggtgaaaa aaattaataa tattttattt 240
aatttaatat gttttaaata ttttaatgtg taattgatta ttattataag ttttggtaat 300
tttgtgtata ttttatattt ggagtatatt ttaattttga ttggttttgt ttttagtatt 360
ttatagttat gtgtggtttg tggttattgt gtgagatatt ttagttttta gttgtaggtt 420
atattgtatg gattttgggg aggaggggtg ttttttgggg gaaggatggg gtttttattg 480
tttttatttt atttgaattt gaaaaagagt ttatttgttt agtgaagggt ttattgtttg 540
gagagttatt gattggtagt tggttgggtt ggttattggt gttgattata gagtttttat 600
attttttagt tatggtagtg ttggttatta gtttattttt ttatgtggtt tttttttatt 660
gtttttagat tgattttaga tgttttggat tgttttgatt gtgtttttag aattttggtt 720
atgtagtatt ttttttagt ttttggaagg gtttttgttg aagttgtgat tttttaggt 780
tttaggagtt aatattagtt taagaaagaa gatttgggag ttaggttagg gaggttgttg 840
tggttattgt gtgtttaggg gttgtgtgtg ttttagtaaa gtatgtggga gttatttatt 900
agattttttg tatgtttatt ttgtggtgat agattttgag agaaatgaat gaaagtaaag 960
atagatttag aaaatggttt ggtatatgtg tttttgttag aagtttagag atttttgag 1020
ttttaggttg tttgttaggt attgggtggg ggagtgagtt tagtttttag ttttaggatt 1080
ttatatatgg gtagaatttg ataatttggg tatttttatt ttattttatt tgttttttgg 1140
gtaagttatt tttttatatg atttagagtt tttatttgta aaatgggaat atttttaatt 1200
gtttttatgt ttaatttgtt tgtttttttg tgagaagtgg gaggtggata atgtttaaga 1260
gttttagatg ttggggaaga tttagttttt ttgtagagag ggatagttta gggtttgggt 1320
tttttttttt tttttgggtt gagaaagttt atggaaagtt ggaataatta tgtatattgt 1380
tatagtaaat ttaatagagt ttttattgtg gtggttttta ggtgattgtg gatgttaagt 1440
aggtaagttt ttggtgaagg ggggagagta gggattagaa gtatttagaa ggggttgaga 1500
gttatgtggg gtttatattg tatttgtagt tgggtttatt ttgattttag gtttaattta 1560
gatgaggaag gattagtagt aattagtgtt atgtgttgtt tttttttagt tttttggggt 1620

atgaatattg tttagttgat gaggggattt tgaaagaatt attatgttta attgttttaa 1680
ttatgtaaat tttgttgata tttttagtta gggaagggtg gttgggtggg agggggttat 1740
ggtggggtta ttttaaagga aaagttttag tttttttatt ttttttatat tttaaggttg 1800
tttttgtggg attttatata gaatagtttg gaagtttggg gttttggttt ttttttttgg 1860
tttgggagtt aggttatggg gttattgttt tatagtaatt atgagggttt aggtttaagt 1920
gtttatatgt tttttatggg gattgttttt tttaaagggt gggttttttt tatttagttt 1980
tttgttttgg ttaaggagga ggttgaaaga gtttgagttg tgtttttttt attttattgt 2040
tgtggtaggt aagtttagat gtgggtaagt ataggtgaag ggaggttagg tgtaggtatt 2100
tttaggtgtg ggtaaattta agtgtggata aatttaggtg tagataatta tagatgtagt 2160
taagtttagg tgttggtaat tttaggtgta ggtaggttta ggtgtaagta agagtaggtg 2220
taggtaagtt taggtgtgtt ttttggggtt tgttatttat ttttttttga ggttggttgg 2280
ataggatttt atggagtagt tggtgaattt gtgtttagtt tgggttttaa gaggttgttt 2340
tagtagaagg gatttattag gttgatttat agaggggata agtattttgt tagttggttt 2400
taaagaggtt gaaatttttt gagttttttt tagggttagt ttttaaattt aggtgttagg 2460
gatttgttta ggagttttgg ttttaggttt taattttttg tttgttattt tttagtattt 2520
tattttgttt tagggaaaag gtgtattttt tttagaattt tagaaaatgt tgaagtttta 2580
ggagagatag ggtttatatt tgtaagtttt tattagttga tattttattt gtgaaagttt 2640
tggggaagag attggttata tggaatagga ttaggagggg agattattgt ttttttttat 2700
tgttgggagt agtgtaaatt ggtttttgga aggtagttta ataatatgta gtaaagtttt 2760
gtaagtttat atttaggaat ttattttgag gagtgaattt tgaagggatg ttaatattga 2820
tatagttttg tgtgttattg ggaagtatag taaataattt aaatgtttag aagtagaggg 2880
ttggatatat taattatggt ttgtttatag gatgaaatat tatgtagtta tttttaaaat 2940
gatagagaaa aaggtataat aattgtatag aattattgtg ttttttatta tatatatatg 3000
tatttgttta atttatttat ggaaaaagtt tagaaatata aggaaatatt agttgtttta 3060
ggatggaggt tttatgggtg gtgtttattt atttttttgt atattttttat atttttttgtt 3120
ttttttaatt attttttatt ataaattaat tttttttttt tttttagaaa atatattttt 3180
tgtatttttt attagtatat tatagttgtg tatattgtgg gggtatatgt gatttttttgg 3240
tgtatgtata taatgtgtat gtttttttaa attttagtaa aaattagtaa aagtagtggg 3300
ggaggtaggg aggaatgaaa atatttgtag ggttaggtgt gatggtttat ggttgtaatt 3360
ttagtatttt gggaggttga ggtaggtaga ttatgaggtt aagagataga gattatttta 3420
gttaatatgg tgaaattttg tttttattaa aaatataaaa attagttggg tatggtagtg 3480
tgtgtttgta gttttagtta tttgagaggt tgaggtagga gaggttgaat ttaggaggtg 3540
gaggttgtag tgagttgaga ttgtgttatt gtattttagt ttgggtgata gagtgagatt 3600
ttattaaaaa aaaaaaaaga aggaaagaaa gaaagaagaa agaaataaag aaagaatatt 3660
tgtataaatt gtgtttgtgt tgataaggga taggaattga gaatggggaa ggaagatgag 3720
atatattttt tttgtttgaa ttagatattt agggaggaat taagaatgga gttttgtttt 3780
tttatgagtg tatttgatga atatgggatt gattttggga aagattgttt ttggttaggt 3840
ttttaaaata ttattgtttt gaattattta gtagtagttt taatttatgg tatttgtaat 3900
tatatttata gtataaattg tttatttttt gtagtgtatt ttttagtttt ttttttttag 3960
aaaatgtaat tggaaatatt aatttatgtt gaatgtgttt gttgtatttg tttatgtttt 4020
attgattatt ttttgggaga agtttggagt tgtgtttggt ttggttttgt gtaattttat 4080
ttatttgttt tgtaaagttg ataagtataa gaagtgaaat gatatttagg tattttaaga 4140
tgtgagttat ttttattttg aattaagtga aaataaaata gattttttat taatttaaaa 4200
tttttttatt gggggtagta gatgttttta gaaaattggt tttggttttg attgataatg 4260
tttggaagat gttttataat ttgagagtgt atgtgtgtgt gtttgggtgt gtgtgtgtaa 4320
atgttggatt tgggaagatg gaagttttta gttaggagaa taagaggata taatattttt 4380
aagtttttta tgatttagag attaagggga tttaatgttt taaaattttt attaagtaga 4440
tagtagtaag agatatgttg tttttagtga tatgtaaagg tatatttgat gggttagtat 4500
ttgttatttt ttttttttgt aagttttgag gttagattta gttatgggtg aaggaattat 4560
agtatttatt gggagtaggt agagtttagg aaagagattt ttttgttatt ttagatgttt 4620
attgtgttag gagtaatttt ttttagtttt tgtagaaagt atatattagt atttatttta 4680
ttgtttttta gaaattgtat ttttaattgt taagtgggga aaaatgtatt tataatgtta 4740
gagttgattt taagaaaata agttaatatt aggttatagt attttttttt tttgttgttg 4800
agattttgtt gttgtgtatt ttgttttttt ttttatggtt tttggttttt gtttttttaa 4860
tttttggttt tttgtttgtt tttgttatta aagtggtaag tataggtatt tttttttttt 4920
attataattt atttattttg tgtttgtttt tttagggtgg gtaatgtttg aaatgtttta 4980
aggtaggggt aatgtttatg gtaggtggtt ttggtttttt tggagatagg gtataggaat 5040
gggtaaattt tttaggtttg gtgaggtgga aagtttgttt tttgggaatt atggttatag 5100
tgtttttttt tgtttgttaa ttattaaaaa gtatattata tgaatatata aagttggaaa 5160
atttatagaa agagttaagt gaagttttgt ttatatattt tttttgattt ttattttttt 5220
gtgttgtgga ttatgtttag tgtttgatgt gtattattat tatttttgtg tgtgtgtgat 5280
agagttttgt tttgttgttt aggttggagt gtaatggtat gattttggtt tattgtaatt 5340
tttgtttttt agatttaagt gatttttttg ttaattagtt gggattatag gtgtttgtta 5400

ttatgtttag ttaattttgt atttttagta gagataaggt tttattatgt tgattaagtt 5460
ggttttaaat ttttgatttt aggtgatttg tttattttag ttttttaaaa tgttgggttg 5520
ggattatagg tgtgagttat tatgtttggt ttgatgtgta ttattttaga tttttgttgt 5580
ttaatagaaa tagtatatgt gttatagata taattttaaa ttgtgtagta gttatttaaa 5640
aaggaaaagt aaaaataaat aagtgaaatt aattttaatt ttataattta tttaatataa 5700
tatatttaaa atatgattgt tttaatatgt agttaatatt aaatatatat taatgaggta 5760
ttttatgttt ttttttattg ttttaagttt ttgaaatttt gttatatatt ttgtttttag 5820
tttttatgtt atagtgaata ttagtgtgtt attttatatt tatagtatgt ttttatttat 5880
tttggttata ttttaggtgt gtaatagttt taggtagtta gggtttatta tgtgggatag 5940
ttttgttttt gaatattggg aagtttgttt ttgtagagtt ggattatatt ggaaatattt 6000
tattatgttt ttttttttaa tttataatgg agatgtttat attttagagt tttgtgttag 6060
ttttgtttat ggttgtatag tttatgattt ggtatgttgt taattggttt aattagtttt 6120
ttaggtagat taataaaaaa gtggaggttt ttaatttttt atttagttta aaatatattg 6180
taataagtat ttatatattg tttatttata tgtaaaagta tttttaggtg attgatttat 6240
ggaagtgaaa gtgttgggtt aaagataggt atgtattttt tgttttgaga ggtaagttag 6300
tatttagtta agttggttat taatttgttt tggggtttgg atgttgtttt tttagaggta 6360
aaagtaaaga gttgggttgg atatttttta tagtttttaa atgtttagtt ttatggttta 6420
aagggtggtg atttttgtta atgtttttgg tggatgtagt tttgggtggg tgggtgggtg 6480
atagttattt gtttttattg gagggtaggt ttggattggg tagataagta aattaattat 6540
atatattttg tttagataag tagaaaatat ttagaaaaga ttttaattgt tttatttgta 6600
aaattgtttt ttggttaatt tttatgaatg tggattattt attaaatgtt attatagaaa 6660
aagagtagag taataaaggg gtttggtagt ttgatttttg tgttggtagt ttttattgtt 6720
atgttgagtt gtggtgaatg tttagggttt tatttgttta ttttttattt ttgttggtgt 6780
gtattttttt tttattttgg tggttttagt aaatattata gtttttgagg gttaggaggt 6840
ttgggttttt agatgtttat atttgatagg tttagtgttt gagtattttg gtgagggatt 6900
tttttggatt atgggagtat tgttagagga ggtttgaggg tagggatttg gatgggtagt 6960
aaaggataga gaatattggg aaaggagagg gtattatttg tgggaattag gtaggtataa 7020
gaggttggtt ttgagttttt gtgtattttt tttgtagata ttggttgtat ttgtttagtt 7080
tttgaaattt agattaggta tttaggtaat gaggggagtt ttttaatata atattttttt 7140
aattgttgtg ttttttggag tgagttggtt aggtgatgtt ggttaaagta ggaaggggag 7200
ttgttgaggt gttttaagat ttttgttgga atttttaggt ttttagataa ttagggtaga 7260
tttggtgatg ggagaaggag atggggatat atatggattt gaagttataa gattttgttt 7320
tttttttttg tttttgttta tttttttaag atgtttattt gttaaataaa gtattttaaa 7380
aagtgtataa gatgtttgta tatagtttag agaatgataa agtagatttt tgtgttttat 7440
tatggggttt ttttttattg tatttttttt tttttagagg tagtattgtt tgatttttgt 7500
ttggtttgtt tttgtttttg tttttttttt tttttttttt tttttttttt tttttttttt 7560
tttttttttt tttttttttt tttgtttttt tttttttttt tttttttttt tttttttttt 7620
tttttttttt tttttttat ttttattttt atttttttga gatagagttt tgtttttgtt 7680
atttaggttg aagtgtagtg gtgttatttt ggtttattgt aatttttatt ttttatgttt 7740
aagtgatttt tttattttag ttttttaaat agttgggatt ataggtaatt gttattatgt 7800
ttggttaatt tttttgtatt tttagtagtg atggggtttt attatgttgg ttaggttggt 7860
tttgaatttt tgatttttag agatttattt attttggttt tttaaagtgt tgggattata 7920
ggtgtgagtt attgtgtttg gtttttggtt tttttttatg ttttatttag tttagttttg 7980
aggtttatat atttttttata tttatttatt tatttatttt taaatggagt tttatttgt 8040
tgtttaggtt ggagtgtagt ggtgtgattt tagtttattg taatttttgt tttttgggtt 8100
taagttattt ttttgtttta gttttttgag tagttgggat tataggtgtt tgttattatg 8160
tttggttaat ttttgtattt ttagtagaga tgaggtttta ttatgttggt taggttggtt 8220
ttgaattttt gattttaggt gatttatttg ttttggtttt ttaaagtgtt gggattatag 8280
atatgagtta ttatgtttag ttatattttt aaataattta ttgagatgaa atttgtatta 8340
tataaaatgg attattttta agtgattaat ttagtggtat ttagtatagt tatgaggtta 8400
tgtaattatt ttgttttttt gtttttattt ttaatattat aaagtaaaat tttttattta 8460
ttaagtagtt tttttttatt tttttttttt gtggttttgt ttttggaaat tattagtttg 8520
tgttttgttt gtatggattt atttattttg gatgttttat ttaaatggaa ttatatatat 8580
gttatttttt atgtttggtt tttattagtt agtattatgt ttttaaggtt tatttatgtg 8640
gtatgtttta gaattttgtt tatttatgtg gttaaataat atttttttgt ttgtataata 8700
tatattataa tttgtttatt tatttattta ttgatggata tgtgagttat ggttattatt 8760
tggttgttat gaatagtgtt gtaatgttat atgtttattt gagtttttgt ttttattttt 8820
tttgggtata tatttagtag taaaattgtt gggttatatg gtaattttat atttaattga 8880
ggaattgtta aattattttt taaagtgatt gtattatttt atatttttat tagtaatgtt 8940
tgaaggtttt aattttttta tatttttgta aatattggtt attatttttt tattttggga 9000
tgtttttaat tataattatt ttagtaggaa tgaagtggta ttttattatg gttttgattt 9060
gttttttttt aatgtttaaa gatgttgggt atttttttat gtgtttgttg gttatttgta 9120
tatttttatt ggagaattgt ttatttaagt tttttgttta gtttttaatt gggttgtttt 9180

tttgttgttg agttggaaga gtttttttat atattttgga tattagattt ttgttatatt 9240
ttgatttgta aatattgtat tttattttat aggttgtggg tttttttttt tttttaataa 9300
tgttttttga tgaatttgat gaatagtttt ttattttaat gaagtttaat ttatttgttt 9360
tttttttttt tgtttatgtt tatggtatta tatttataaa tttattgatt atgaagataa 9420
tgaaaattta tatatatatt ttttttaaga gttttatggt tttaggtttt ataatttagt 9480
ttgatttatt ttgagttaat atttatagtt agtgtgaagt aggggtttaa atttattttt 9540
ttgtatgtgg atatttaatt ttttaagtat tattagttga taagattgtt ttttttttta 9600
ttgaatggtt ttagtatttt tgttaaaaat tatttggata tagatatatg gatttatttt 9660
tgaaatttta attttatttt attggtttgt atgtttgttt ttatgttagt attatatttt 9720
tttgattatt gatgttttga aattttgaaa ttagaaaata tgagtttttt aattttgttt 9780
ttttttttaa gattgttttg ttggttatgt gtggggatta tgtttgtaat tttagtattt 9840
tgggaggttg aggtgggtag attatttgag gttaggagtt ggaggttagt ttggttaata 9900
tggtgaaatt ttatttttat taaaaataaa aaaaattagt taggtatagt ggtatatgtt 9960
tgtagtttta gttattttag aggttgaggt ataagaatta tttgaatttg ggaggtagag 10020
gttgtagtga gttgagatta tgttattgta ttttagtttg ggtaatagag taagatttta 10080
ttttaaaata aaaaaataaa aaaaaaaatt agattgtttt gtttatttag tttattgtaa 10140
tttatgaata taggatgttt tttagaattg tgtataaaaa atatttttaa taggtggata 10200
tggatgaatt aaggtgtaaa atgagaatta ttaaatatat atttgtaagt tatttttttt 10260
aaaaggtatt agagttaaga tttttaaaga tttgtattta ttattattgt agatgattat 10320
ttagatgttg tgggtagata tattgtttta taaaggtata agaatggatt ttgatgatgg 10380
tttgggaatg tattggttta atatttattt tgattattga tgttttgaaa ttttgaaatt 10440
agaaaatatg agttttttaa ttttgttttt ttttttaaga ttgttttgtt ggttatgtgt 10500
ggggattatg tttgtaattt tagtattttg ggaggttgag gtgggtagat tatttgaggt 10560
taggagttgg aggttagttt ggttaatatg gtgaaatttt atttttatta aaaataaaaa 10620
aaattagtta ggtatagtgg tatatgtttg tagttttagt tattttagag gttgaggtat 10680
aagaattatt tgaatttggg aggtagaggt tgtagtgagt tgagattatg ttattgtatt 10740
ttagtttggg taatagagta agattttatt ttaaaataaa aaaataaaaa aaaaaattag 10800
attgttttgt ttatttagtt tattgtaatt tatgaatata ggatgttttt tagaattgtg 10860
tataaaaaat atttttaata ggtggatatg gatgaattaa ggtgtaaaat gagaattatt 10920
aaatatatat ttgtaagtta ttttttttaa aaggtattag agttaagatt tttaaagatt 10980
tgtatttatt attattgtag atgattattt agatgttgtg ggtagatata ttgttttata 11040
aaggtataag aatggatttt gatgatggtt tgggaatgta ttggtttaat atttatt 11097

<210> 5
<211> 11097
<212> DNA
<213> Artificial Sequence

<220>
<223> chemically treated genomic DNA (Homo sapiens)

<400> 5

agtggatatt ggattaatat atttttaagt tattattaga gtttattttt atatttttat 60
aaaataatat gtttgtttat aatatttgaa tagttatttg taataatggt ggatgtaagt 120
ttttaaggat tttagtttta gtatttttta agagaaatgg tttgtaagta tgtatttaat 180
ggtttttatt ttgtatttta atttatttat atttatttgt tgaagatatt ttttgtgtat 240
ggttttgaaa gatattttgt atttatgaat tgtaatgggt taaatggata gagtaatttg 300
gttttttttt ttgttttttt attttgagat ggagttttgt tttgttgttt aggttggagt 360
gtagtggtat gattttggtt tattgtagtt tttgtttttt aggtttaagt gatttttgtg 420
ttttagtttt tggagtagtt gggattatag atatgtgtta ttatgtttgg ttaatttttt 480
ttatttttag tagagatagg gttttattat gttggttagg ttggtttttg atttttgatt 540
ttaagtgatt tgtttgtttt agttttttaa agtgttggga ttataaatat gatttttgta 600
tatggttgat aaagtaattt tgaaaaaaag aataaagtta gaggatttat atttttttgat 660
tttagaattt taaagtgtta gtaattaaag tggatattgg attaatatat ttttaagtta 720
ttattagagt ttattttttat atttttataa aataatatgt ttgtttataa tatttgaata 780
gttatttgta ataatggtgg atgtaagttt ttaaggattt tagttttagt attttttaag 840
agaaatggtt tgtaagtatg tatttaatgg tttttatttt gtattttaat ttatttatat 900
ttatttgttg aagatatttt ttgtgtatgg ttttgaaaga tattttgtat ttatgaattg 960
taatgggtta aatggataga gtaatttggt tttttttttt gtttttttat tttgagatgg 1020
agttttgttt tgttgtttag gttggagtgt agtggtatga ttttggttta ttgtagtttt 1080
tgttttttag gtttaagtga tttttgtgtt ttagtttttg gagtagttgg gattatagat 1140
atgtgttatt atgtttggtt aatttttttt attttttagta gagatagggt tttattatgt 1200

tggttaggtt ggtttttgat ttttgatttt aagtgatttg tttgttttag tttttttaaag 1260
tgttgggatt ataaatatga tttttgtata tggttgataa agtaattttg aaaaaaagaa 1320
taaagttaga ggatttatat tttttgattt tagaatttta aagtgttagt aattaaagaa 1380
gtatggtatt agtataagga tagatatgta ggttagtgga gtaagattga gattttagaa 1440
ataagtttat atatttatgt ttaagtgatt tttagtaaag gtgttaagat tatttaatgg 1500
gggaaaagat agttttgtta attaatagtg tttggaaaat tggatattta tatgtaaaag 1560
aatgaatttg gatttttatt ttatattagt tataaatatt aatttaaaat ggattagatt 1620
aaattgtaag atttaaaatt ataaaatttt taaaagaaat atatgtgtga atttttatta 1680
tttttatgat tgatggattt atagatatga tattataagt atgaataaga aaagaaaaag 1740
taggtaagtt gaattttatt aaaatgaaaa attgtttatt aaatttatta aaggatatta 1800
ttaagaaagg aaaaaaattt ataatttata gaatggaata tgatatttgt aaattaaaat 1860
atgataaggg tttaatattt agaatatata aaagaatttt tttaatttaa taataaaaag 1920
ataatttgat taaaaattgg gtaaaggatt tgaatagata atttttttaat gaagatatat 1980
aaatggttaa taaatatatg aaaagatatt taatattttt agatattagg gaaaagtaaa 2040
ttaaaattat gatgaggtgt tattttattt ttattaggat gattataatt aaaaatattt 2100
taaaataaaa aaatagtaat tagtgtttgt aaggatgtgg aggaattgga atttttagat 2160
attgttggtg aaagtgtaaa atggtatagt tattttggaa aatgatttgg tagtttttta 2220
gttaaatgta gaattattat atgatttagt aattttattg ttaggtatat atttaagaga 2280
aatgaaaata gagatttaaa taaatatgta gtattgtagt attatttata atagttaaat 2340
ggtggttata gtttatatgt ttattagtgg atagatggat aaataaattg tggtatatgt 2400
tatataaata aaagaatatt atttagttat ataaatgaat gaagttttga agtatgttat 2460
atgggtgaat tttggaaatg tgatgttaat tgatagaagt taggtataaa aggtgatata 2520
tgtatgattt tatttagatg aaatatttag aatagataaa tttatataga taaagtataa 2580
attagtggtt tttaggggta aggttatagg gagaggaaat ggggaggaat tgtttaatgg 2640
gtagggggtt ttattttgta gtgttggaag taaggataga gagataaggt agttgtatga 2700
ttttatgatt gtattaaata ttattgaatt ggttatttga aaatggttta ttttatgtga 2760
tataaatttt attttaataa attatttaaa aatatggttg ggtatggtgg tttatgtttg 2820
taattttagt attttgggag gttgaggtgg gtggattatt tgaggttagg agtttgagat 2880
tagtttggtt aatatggtga aattttgttt ttattaaaaa tataaaaatt agttaggtat 2940
ggtgataggt gtttgtaatt ttagttattt gggaggttga ggtaggagaa tagtttgaat 3000
ttaggaggta gaggttgtgg tgagttgaga ttatattatt gtattttagt ttgggtgata 3060
gagtgagatt ttatttgaaa ataaataaat aaataaatat aaaaaatatg taaattttaa 3120
aattaaatta aatgaaatat aaagaaaagt taggggttag gtgtagtggt ttatatttgt 3180
aattttagta ttttgggagg ttaaggtggg tggatttttg gaggttagga gtttgagatt 3240
agtttggtta atgtggtgaa attttattgt tattaaaaat ataaaaaaat tagttaggtg 3300
tggtggtagt tgtttgtaat tttaattatt tgggaggttg aggtaggaga attgtttgaa 3360
tatgggaggt gaaggttgta gtgagttgag atagtattat tgtattttag tttaggtgat 3420
aagagtaaaa ttttgtttta aaaaaataaa aataaaaata gaaaagaaaa gaaaagaaaa 3480
gaaagaagga aggaaggaaa gaaaaagaag aaatgaaggg aagggaagaa ggaaggaagg 3540
gagggaggaa gggagggaaa aagagagaaa gaaagagtaa aggtaggaat gagttaggta 3600
gaagttaggt agtgttgttt ttgggaggga aggagtgtag tgggaaaggg ttttatggta 3660
aaatataagg atttgtttta ttatttttta aattatgtgt agatatttta tatatttttt 3720
aaaatgtttt atttagtaat aaaatatttt aaaagaataa ataaaaatag gagagagaag 3780
tagaattttg tagttttgga tttatgtgtg tttttatttt ttttttttat tattgggttt 3840
attttgattg tttgggaatt tgaggatttt agtaaggatt ttggggtatt ttaatagttt 3900
ttttttttgt tttggttaat attatttggt tagtttattt tgaaaagtat agtagttggg 3960
aaggtgttgt gttaaggagt tttttttatt gtttgagtgt ttggtttggg ttttagaggt 4020
taggtaggtg tagttagtgt ttgtaggaga gatgtgtaga ggtttagggt tagttttttg 4080
tgtttgtttg gtttttatag gtagtatttt tttttttttt gatgtttttt gttttttgtt 4140
atttatttag gtttttgttt ttgagttttt tttggtagtg tttttatggt ttagggaagt 4200
tttttattag agtgtttagg tattaggttt gttaggtgtg ggtatttggg gatttaagtt 4260
ttttggtttt tagggattgt gatgtttgtt ggggttatta gggtggggaa agggtgtatg 4320
ttagtaggag tgaggggtag gtgggtgaaa ttttaggtat ttattatagt ttagtgtgat 4380
ggtggaggtt gttagtatgg gaattagatt gttgggtttt tttgttgttt tgtttttttt 4440
ttatgataat gtttaatgaa tgatttatat ttatgaaaat tagttaaaga gtaattttat 4500
aaataaaata attggggttt tttttgagtg tttttttgttt atttagataa ggtgtgtgtg 4560
gttgatttgt ttatttgttt aatttaggtt tgttttttag tgggagtagg tagttgttat 4620
ttatttattt atttaagatt atgtttatta aaggtattgg taggaattat tattttttgg 4680
gttatggaat tgagtattta ggaattatgg agggtattta gtttaatttt ttgtttttat 4740
ttttaaggga ataatgttta aattttaagg taaattggtg gttggtttgg ttgagtattg 4800
gtttgttttt tagagtaaag ggtgtatgtt tgtttttgat ttagtatttt tatttttatg 4860
gattgattat ttggaaatat ttttgtatgt aaataggtaa tgtatggatg tttgttgtag 4920
tgtattttaa gttaagtgaa aaattggaaa tttttatttt tttgttagtt tatttggggg 4980

attgattaaa ttaattaatg atgtattaaa ttatggatta tgtaattatg aataagatta 5040
atatagaatt ttaaagtgta aatgttttta ttatgaattg aaaaaaaaag tgtagtaaaa 5100
tatttttgat gtgatttagt tttataggaa taaatttttt gatatttaag agtagagttg 5160
ttttgtatgg tagattttgg ttatttgggg ttgttgtgta tttgagatgt ggttagggtg 5220
aatggagata tgttgtgagt gtgagatggt atgttaatgt ttattgtgat atgagagttg 5280
aaaatgaggt gtgtggtagg atttaaaaga tttagaataa taaagaaaga tataaaatat 5340
tttattagta tgtgtttggt attgattata tgttgaaata attatatttt agatatattg 5400
tgttaaatga attatagaat taaagttaat tttatttatt tgtttttatt tttttttttt 5460
aagtggttat tatatagttt gaaattatgt ttgtggtgtg tgtgttattt ttattaggta 5520
gtagaggttt agaataatat atattaggtt gggtgtggtg gtttatgttt gtaattttag 5580
tttagtattt tgggaggttg aggtgggtgg attatttgag gttaggagtt tgagattagt 5640
ttgattaata tggtgaagtt ttgtttttat taaaaatata aaattagttg ggtgtggtgg 5700
tgggtatttg taattttagt tagttggtag gagaattgtt tgaatttggg aggtggaggt 5760
tgtggtgagt tgagattatg ttattgtatt ttagtttgag taatagagta agattttgtt 5820
atatatatat aaaaataata ataatatatg ttaaatatta agtatggttt atagtatggg 5880
agagtaggga ttaggaggag tgtgtaagta ggattttatt tggttttttt gtggggtttt 5940
ttaattttat atatttatgt agtatgtttt ttaatgatta ataaataaaa aagggtattg 6000
tggttatgat ttttgaggag taagtttttt attttattaa gtttgaagaa tttgtttatt 6060
tttgtgtttt gtttttagga gagttagggt tatttgttat gagtgttatt tttattttaa 6120
ggtattttag gtattattta ttttggggga gtagatatag gatggataag ttgtgatgag 6180
ggaggaggat atttgtgttt gttattttaa tggtaggggt agataggggg ttagggattg 6240
ggggagtggg agttagaagt tgtgagggag gggataaggt gtatagtgat aaggttttag 6300
tagtggggga ggggagtgtt gtggtttgat gttaatttgt ttttttaaag ttagttttag 6360
tattgtaaat gtattttttt ttatttagta attgaagata tagtttttga aaagtagtga 6420
gatagatatt aatatgtgtt ttttataaga ggttgggaaa attgtttttg gtgtgatggg 6480
tatttgaggt aatagaaaaa tttttttttt ggattttgtt tgtttttaat ggatgttgta 6540
atttttttat ttatggttga gtttaatttt agagtttgta aaagaggagg atggtagata 6600
ttgatttatt agatatgttt ttgtatatta ttgagggtaa tatgtttttt gttattgttt 6660
atttaatgga ggttttaaga tgttaaattt ttttaatttt taggttatgg aaggtttggg 6720
ggtattgtgt ttttttattt ttttggttga gggtttttat ttttttaggt ttagtattta 6780
tatatatata tttagatata tatatatata tttttaaatt ataggatatt ttttaaatat 6840
tgttaattaa agttaaagtt aattttttaa aaatatttat tgtttttagt aggaggattt 6900
taggttgata gaaaatttgt tttgttttta tttagtttag aatgaagatg atttgtattt 6960
taaaatgttt gagtgttatt ttattttttg tgtttattag ttttatagag taaataaatg 7020
aggttgtata gagttaaatt aaatataatt ttaaattttt tttagaaaat aattaataaa 7080
atataaatag gtatagtaaa tatatttaat atgagttgat atttttaatt atattttttg 7140
ggggaggagg gttaaaaaat atattgtaga gaatagataa tttatgttat aaatataatt 7200
gtaaatgtta tgaattaaaa ttgttgttgg atagtttaaa ataatggtgt tttgagaatt 7260
tgattaaagg tagttttttt tagaattagt tttgtattta ttaagtgtat ttatagagag 7320
gtagggtttt gtttttaatt tttttttaaa tatttgattt agatagagaa aatgtgtttt 7380
attttttttt tttattttta gtttttattt tttgttaatg taaatataat ttatgtaaat 7440
attttttttt tgtttttttt tttttttttt tttttttttt tttttttttt ttgatggagt 7500
tttattttgt tgtttaggtt ggagtgtagt ggtgtagttt tggtttattg taatttttat 7560
tttttgggtt taattttttt tgttttagtt ttttgagtag ttgggattat aggtgtgtat 7620
tattatgttt agttaatttt tgtattttta gtagagatag ggtttattta tgttggttag 7680
gatggttttt atttttttgat tttgtgattt gtttgtttta gtttttttgaa gtgttgggat 7740
tatagttatg agttattgtg tttggttttg taaatatttt tatttttttt tatttttttt 7800
attatttttg ttaatttttg ttaaggttta aaaaaatata tatattgtat atatgtatta 7860
aaaaattata tgtattttta taatatgtat aattatgatg tattaataaa aagtataaaa 7920
gatatatttt ttgggaaaaa aaggaaaatt aatttgtgat agaaaatgat taaagaagat 7980
aaaaaatgta gaaatgtata aaagaataaa taaatattat ttataaggtt tttattttaa 8040
aataattgat atttttttgt atttttaggt tttttttgtg aatagattag ataggtgtat 8100
gtgtgtgtaa tgaaaaatat aataattttg tatggttatt atgttttttt ttttgttatt 8160
ttaaaagtga ttgtgtgatg ttttatttta tggatagatt atgattgatg tgtttagttt 8220
tttgtttttg ggtgtttggg ttgtttgttg tgttttttgg tagtatataa agttgtgtta 8280
atgttggtat ttttttagga tttgtttttt aggatgggtt tttaggtgtg gatttgtagg 8340
gttttgttgt atgttgttga attgtttttt agaaattagt ttgtattgtt tttagtagtg 8400
agagggagta gtaatttttt tttttgattt tgttttgtgt aattaatttt ttttttaagg 8460
tttttatagg tgaaatatta gttgataaag gtttataagt ataaattttg ttttttttag 8520
aattttagta ttttttgggg ttttaaggaa ggtgtatttt ttttttgaag taaagtaaga 8580
tgttaaggaa tgataggtaa gaggttgagg tttggagttg gggtttttga gtagattttt 8640
ggtatttgga tttagaagtt ggttttgagg gaagtttaga gggttttagt ttttttgaga 8700
ttagttgata gagtatttat tttttttgtg aattaatttg gtgggttttt tttgttgaga 8760

tagtttttg gaatttaggt tgaatatagg tttattagtt gttttatagg gttttgttta 8820
gttagtttta agaggagatg agtaataggt tttagaaagt atatttgagt ttgtttgtat 8880
ttgtttttat ttgtatttga gtttatttgt atttgaggtt attaatattt gagtttaatt 8940
gtatttgtgg ttatttgtat ttgagtttgt ttatgtttga gtttatttat atttgaggat 9000
atttgtattt gatttttttt tatttgtgtt tatttgtatt tgagtttatt tgttatagta 9060
gtggaatgga gagggtatag tttaggtttt tttagttttt tttttggtta gggtagggag 9120
ttgggtgagg agggtttatt ttttaagagg agtagttttt atgaggagta tgtgagtatt 9180
tgggtttggg tttttatgat tgttgtgaag tgatggtttt atgatttgat ttttaggtta 9240
gaaaaggaag ttagggtttt aagtttttag gttgttttgt gtggaatttt ataaaggtag 9300
ttttaggatg tgagagaggt aggggagtta gagttttttt tttgaagtgg ttttgttatg 9360
gttttttttt atttagttgt ttttttttgg ttggaaatgt tagtagggtt tgtataattg 9420
agataattgg atataatggt tttttagaa ttttttttatt agttgggtag tgtttgtgtt 9480
ttggggagtt gggagaaggt ggtatatggt attaattatt gttaattttt ttttatttaa 9540
gttgggtttg aggttagggt gaatttagtt gtaaatgtag tgtgagtttt atatgatttt 9600
tagttttttt tgagtgtttt taatttttgt tttttttttt ttattaggag tttgtttgtt 9660
tggtatttat agttatttga aaattattat ggtgggagtt ttgttggatt tgttgtaata 9720
gtatgtgtgg ttattttagt tttttatgag tttttttagt ttaggaggaa agaaaaaatt 9780
taaattttga attatttttt tttgtagagg ggttgggttt tttttagtat ttgaggtttt 9840
tgggtattgt ttgtttttg tttttatag aggagtagat agattggatg taagggtaat 9900
taggggtgtt tttattttat agatgaggat tttgagttat gtgagagggt ggtttgttta 9960
gagagtaggt ggggtggagt gggagtgttt aggttgttaa gttttgttta tatgtagagt 10020
tttgaagttg gaggttgggt ttattttttt atttaatgtt tggtagatag tttggggttt 10080
agggaatttt tgaattttg atggaagtat atgtgttagg ttattttttg ggtttgtttt 10140
tgtttttatt tgtttttttt agggtttgtt attgtagagt gggtgtgtaa agggtttggt 10200
gagtggtttt tatgtgtttt gttggagtgt gtatagtttt tgggtgtata atgattgtag 10260
tagtttttt ggtttggttt ttgagttttt tttttgaat tggtgttggt ttttgaggtt 10320
tgagagagtt atggttttag tgggaatttt tttggggggtt gagggagggt gttatgtggt 10380
tggaattttg aaggtatagt tagggtaatt tggggtattt gaaattagtt tgggagtagt 10440
ggggaaagat tatatgggga agtgagttga tggttggtat tgttatggtt gggaggtgtg 10500
gaggttttgt ggttagtatt aatggttagt ttgattggtt gttagttagt ggttttttag 10560
gtagtgggtt ttttattaga taaataagtt tttttttagg tttagataag atggaaataa 10620
tgggagtttt attttttttt aagagaatat tttttttttt tagggtttat gtagtgtggt 10680
ttgtaattgg gagttgagat gttttatata gtggttatag gttatgtgtg gttatggagt 10740
gttggaaata gagttagtta gaattgagat atgttttgaa tgtaaaatat atatgggatt 10800
gttaagattt gtgataataa ttgattgtat attgaaatat ttagaatatg ttgggttgaa 10860
tagaatgtta ttaatttttt ttatttgttt ttatttattt tttaaaattg attattagaa 10920
aattttaaat tggtttagtt tatgtttgta attttagtat tttgggaggt tgaggtaggt 10980
ggattatttg aggttaggag tttgagatta gtttggttaa tatggtgaaa ttttattttt 11040
attaaaaata taaaaaataa ttggttatgg tggtgggtgt ttgtaatttt agttatt 11097

<210> 6
<211> 22
<212> DNA
<213> Artificial Sequence

<220>
<223> primer_1 for amplification of bisulfite treated MLSN1 fragment con-
taining CpG site 1

<400> 6

tagagttttg aagttggagg tt 22

<210> 7
<211> 22
<212> DNA
<213> Artificial Sequence

<220>
<223> primer_2 for amplification of bisulfite treated MLSN1 fragment con-
taining CpG site 1

<400> 7

tcacttcccc atataatctt tc                                                    22

<210> 8
<211> 18
<212> DNA
<213> Artificial Sequence

<220>
<223> detection oligonucleotide for MLSN1 CpG site 1 methylated

<400> 8

gggtaattcg gggtattt                                                        18

<210> 9
<211> 18
<212> DNA
<213> Artificial Sequence

<220>
<223> detection oligonucleotide for MLSN1 CpG site 1 unmethylated

<400> 9

gggtaatttg gggtattt                                                        18